# EUROPEAN PATENT APPLICATION

(11) **EP 3 988 559 A1**
(43) Date of publication of application: **27.04.2022**
(21) Application number: 20826764.1
(22) Date of filing: 22.06.2020
(51) Int. Cl.: C07H 19/16, A61K 31/7076, A61P 9/00, A61P 37/00, A61P 17/00

(54) **2-BENZYLIDENE HYDRAZINOADENOSINE COMPOUNDS HAVING A2A ADENOSINE RECEPTOR AGNOSTIC ACTIVITY**

(30) Priority: 21.06.2019 CN 201910542124
(71) Applicant: Academy Of Military Medical Sciences, Haidian District Beijing 100850 (CN)
(72) Inventor: ZHONG, Wu, Beijing 100850 (CN); ZHANG, Min, Beijing 100850 (CN); ZHOU, Xinbo, Beijing 100850 (CN); FAN, Shiyong, Beijing 100850 (CN); LI, Song, Beijing 100850 (CN)
(74) Representative: Böhm, Brigitte
(86) International application number: PCT/CN2020/097442
(87) International publication number: WO 2020/253872

(57) **Abstract**

2-Benzylidene hydrazinoadenosine compounds having A_{2A} adenosine receptor agonistic activity, represented by a general Formula (I) and pharmaceutical compositions containing the same. The compounds and compositions can act as A_{2A} adenosine receptor agonist to serve as medicaments.

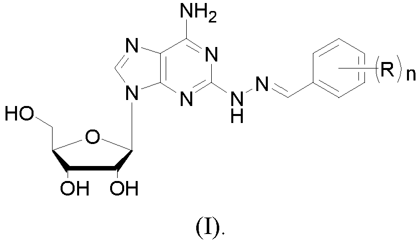

## Description

The present application is based on and claims the benefit of priority from Chinese application No. 201910542124.5, filed on June 21, 2019, the disclosures of which are incorporated herein by reference in its entirety.

### Technical Field

The present application belongs to the technical field of medicine, specifically relates to a 2-benzylidene hydrazinoadenosine compound having A_{2A} adenosine receptor agonistic activity and a pharmaceutical composition containing the same. These compounds and compositions can be used as a medicament.

### Background Art

For drugs for the treatment of central nervous system diseases, one of the main reasons for the failure of their development and related research lies in the obstruction of the blood-brain barrier (BBB), which prevents a drug from being delivered to the central nervous system and accumulating in the brain to reach an effective dose to produce a corresponding therapeutic effect. Therefore, a key factor for the successful development of drugs that target the center nervous system is to overcome the blood-brain barrier. Research on methods of opening the blood-brain barrier has become a hot spot for intracerebral drug delivery. Drug delivery across the blood-brain barrier has been a challenging research field in the past few decades. Researchers have made considerable efforts to develop various drug delivery systems, and a series of strategic studies have revealed that the delivery of drugs and contrast agents across the blood-brain barrier are very difficult. However, for the treatment of central nervous system diseases, such as brain tumors, strokes, trauma and neurodegenerative diseases, as well as nerve agent poisoning, the demand for effective therapeutic drugs has increased dramatically, and the design of drugs that can penetrate the blood-brain barrier is particularly important. Therefore, it is necessary to develop an efficient blood-brain barrier disruption (BBBD) strategy, which has less nerve damage, can deliver drugs of larger molecular weight, and has better pharmacokinetic characteristics.

The BBB restricts the entry of molecules into the brain through two main structural features. First of all, the tight junctions (TJs) structure seals endothelial cells, resulting in low permeability of blood molecules through the BBB. On the other hand, compared with peripheral vascular endothelial cells, there are few transport pathways between cerebral vascular endothelial cells, but the expression level of active efflux transporters, such as P glycoprotein (P-gp) on brain capillary endothelial cells (BCECs), is very high. Considering the key role of TJs in restricting the entry of molecules into the brain (HUBER J D et al., Trends Neurosci, 2001, 24(12): 719-25), reversibly changing the tightness of TJs may be a feasible way to up-regulate the permeability of BBB. Temporary opening of TJs is a feasible way to deliver drugs into the brain because of the high passing efficiency and less molecular weight restriction for therapeutic drugs. Bynoe et al. recently demonstrated that the specific activation of A_{2A} adenosine receptor (A_{2A}AR) on BCECs of mouse can promote the absorption of drug in the brain (CARMAN AJ et al., J Neurosci, 2011, 31(37): 13272-80). Further studies have shown that activation of A_{2A} adenosine receptor can up-regulate BBB permeability and temporarily increase the intercellular space of brain capillary endothelial cells. Studies have shown that the A_{2A}AR signaling pathway modulates cytoskeletal elements by regulating intracellular actin, resulting in cell morphology contraction, destruction of TJs integrity, and increase of barrier permeability (SOHAIL MA et al., Hepatology, 2009, 49(1): 185-94). Therefore, these studies have greatly expanded the potential application fields and development space of A_{2A}AR agonists. The development of efficient A_{2A}AR agonists is of great significance to the study of strategies for the blood-brain barrier disruption (patent application: CN200980117596.0).

At the same time, due to the widespread distribution of A_{2A}AR in the human body, A_{2A}AR agonists are recommended for the treatment of various pathological diseases. Adenosine mediates A_{2A}AR to produce potential immunosuppressive and blood pressure lowering effects. One of the main potential therapeutic effects of A_{2A}AR agonists is anti-inflammatory and immunosuppressive effects by regulating the activity of neutrophils, macrophages and T lymphocytes (DE LERA RUIZ M et al., J Med Chem, 2014, 57(9): 3623-50; VARANI K et al., FASEB J, 2010, 24(4): 1192-204). From the perspective of cell signaling pathways, the activation of A_{2A} adenosine receptors reduces the NF-kB pathway, reduces inflammatory cytokines such as tumor necrosis factor α (TNF-α) and Interleukin-1 Beta (IL-1β), IL-8, IL-6, and inhibits the release of matrix metalloproteinase-1 (MMP-1) and MMP-3 (HASKO G, etc., Nat Rev Drug Discov, 2008, 7(9): 759-70). Therefore, selective agonists have been developed to treat related diseases, such as allergic rhinitis, asthma, and chronic obstructive pulmonary disease. However, the systematic use of A_{2A}AR agonist for anti-inflammatory drugs is limited, because when the activation of A_{2A}AR produces anti-inflammatory effects, it stimulates the heart and blood vessels to cause potent hypotensive activity. On the other hand, A_{2A}AR agonists are powerful vasodilators and have been used as diagnostic reagents for cardiac pharmacologic stress tests (patent application: CN200580033215.2). Although A_{2A}AR agonists as powerful vasodilators can produce systemic side effects, it is reported that low doses may not produce significant cardiovascular side effects. In addition, further potential therapeutic applications of A_{2A}AR agonists are the treatment of psychosis and Huntington's disease (AKKARI R et al., Curr Top Med Chem, 2006, 6(13): 1375-99; BOSCH MP et al., J Med Chem, 2004, 47(16): 4041-53). A_{2A}AR agonists have been shown to have neuroprotective effects on neurodegenerative disease models by reducing the release of excitatory neurotransmitters, apoptosis and inflammation (MULLER C E et al., Biochim Biophys Acta, 2011, 1808(5): 1290-308; RIVERA-OLIVER M, etc., Life Sci, 2014, 101(1-2): 1-9).

Although the aforementioned A_{2A}AR agonists have been increasingly developed, only one receptor agonist, Regadenoson (an adenosine analog), is approved as a coronary vasodilator in the United States. Regadenoson is a selective A_{2A} adenosine receptor agonist jointly developed by CV Therapeutics and Astellas, which has been marketed in the United States and Europe. It is mainly used as a coronary vasodilator for myocardial perfusion imaging. Therefore, there is still a need in the art for novel, effective A_{2A} receptor agonists that optionally have one or more physiological and/or physicochemical advantages, and it is important to further synthesize and test other A_{2A} receptor agonists in order to develop new and improved therapeutic agents.

### Contents of the Present Application

The purpose of the present application is to find and develop a new type of small molecule agonist acting on A_{2A} adenosine receptor, which can agonize A_{2A} adenosine receptor, so that on the one hand, the purpose for the prevention or treatment of a human pathological condition or symptom is achieved, in which the prevention or treatment of a human pathological condition or symptom involves the activity of A_{2A} adenosine receptor and the activation of this activity is required; on the other hand, the purpose of increasing the permeability of blood-brain barrier of the subject receiving the therapeutic drug is also achieved. In the present application, it has been found through research that the compound with the following general Formula I can act on A_{2A} adenosine receptor and is an A_{2A} adenosine receptor agonist, so it can be used for the above two purposes. The present application is completed based on the above findings.

Therefore, the first aspect of the present application provides a compound represented by the general Formula (I), a stereoisomer, a pharmaceutically acceptable salt, a pharmaceutically acceptable hydrate or solvate, or a pharmaceutically acceptable ester thereof, 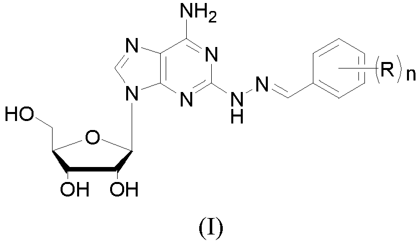 wherein,
n is 1, 2, 3, 4 or 5;
R represents a substituent attached to the benzene ring, and each R is independently selected from the group consisting of hydrogen, halogen, cyano, benzyloxy, halogenated benzyloxy, C₁₋₆ alkyl, halogenated C₁₋₆ alkyl, C₁₋₆ alkoxy, hydroxyl, C₁₋₆ alkylamino, di(C₁₋₆ alkyl)amino, anilino, diphenylamino, phenylamino,-NHC(O)R¹⁰, aryl, heteroaryl, cycloalkyl, and heterocycloalkyl, where R¹⁰ is C₁₋₆ alkyl.

The second aspect of the present application provides a method for preparing the compound represented by the general Formula (I), a stereoisomer, a pharmaceutically acceptable salt, a pharmaceutically acceptable hydrate or solvate, or a pharmaceutically acceptable ester as described in the first aspect of the present application, comprising: 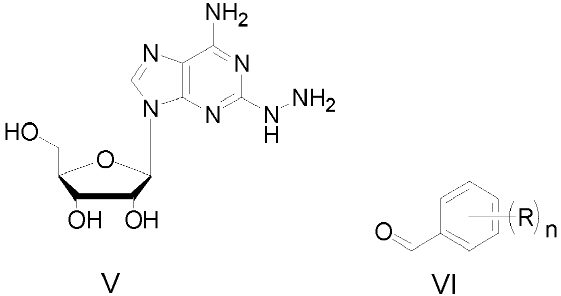 reacting a compound represented by Formula V with a substituted benzaldehyde represented by Formula VI to obtain the compound represented by general Formula (I), wherein the definitions of R and n are the same as those described in the first aspect of the present application.

In some embodiments, in the method described in the second aspect of the present application, the compound represented by Formula V reacts with the substituted benzaldehyde represented by Formula VI in a methanol solution under microwave heating at 70°C to 90°C.

In some embodiments, in the method described in the second aspect of the present application, the compound represented by Formula V is produced by the hydrazinolysis of a compound represented by Formula IV with hydrazine hydrate at 40°C to 60°C.

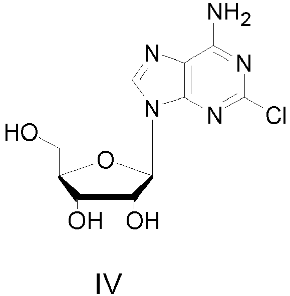

In some embodiments, in the method described in the second aspect of the present application, the compound represented by Formula IV is produced by the ammonolysis of a compound represented by Formula III in a solution of ammonia in methanol at 90°C to 110°C.

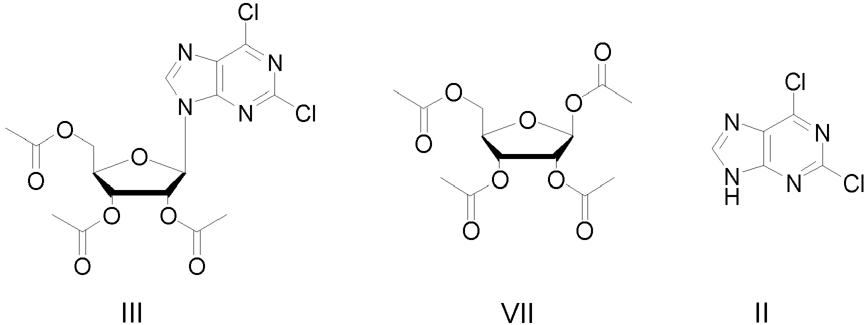

In some embodiments, in the method described in the second aspect of the present application, the compound represented by Formula III is produced by a substitution reaction of a compound represented by Formula VII with a compound represented by Formula II in the presence of tin tetrachloride as a catalyst at 110°C to 130°C.

The third aspect of the present application provides a pharmaceutical composition, which comprises at least one of the compound, a stereoisomer, a pharmaceutically acceptable salt, a pharmaceutically acceptable hydrate or solvate, or a pharmaceutically acceptable ester thereof as described in the first aspect of the present application, and one or more pharmaceutically acceptable carriers or excipients.

The fourth aspect of the present application provides use of the compound, a stereoisomer, a pharmaceutically acceptable salt, a pharmaceutically acceptable hydrate or solvate, or a pharmaceutically acceptable ester thereof as described in the first aspect of the present application, or the pharmaceutical composition as described in the third aspect of the present application in the manufacture of a medicament as an A_{2A} adenosine receptor agonist, or
in the manufacture of a medicament for the prevention and/or treatment of a human pathological condition or symptom, wherein the human pathological condition or symptom is related to the activity of A_{2A} adenosine receptor, and the prevention or treatment of the human pathological condition or symptom requires the activation of A_{2A} adenosine receptor.

According to some embodiments of the present application, the human pathological condition or symptom described in the present application is selected from the group consisting of: autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, foundrymen's disease, glaucoma, ocular hypertension, spinal cord injury, myocardial infarction, and acute myocardial infarction.

The fifth aspect of the present application provides use of the compound, a stereoisomer, a pharmaceutically acceptable salt, a pharmaceutically acceptable hydrate or solvate, or a pharmaceutically acceptable ester thereof as described in the first aspect of the present application, or the pharmaceutical composition as described in the third aspect of the present application in the manufacture of a medicament for diagnosing a human abnormal myocardial perfusion, or in manufacture of a medicament as a coronary vasodilator.

The sixth aspect of the present application provides use of the compound, a stereoisomer, a pharmaceutically acceptable salt, a pharmaceutically acceptable hydrate or solvate, or a pharmaceutically acceptable ester thereof as described in the first aspect of the present application, or the pharmaceutical composition as described in the third aspect of the present application in the manufacture of a medicament for increasing the permeability of blood-brain barrier of a subject receiving a therapeutic drug, wherein the subject benefits from the increased permeability of blood-brain barrier for delivering the therapeutic drug across the blood-brain barrier.

According to some embodiments of the present application, in the use described in the sixth aspect of the present application, the therapeutic drug is selected from the group consisting of: drug for treating disease or disorder of central nervous system, antidote to nerve agent, and drug for treating glioma.

The seventh aspect of the present application provides a pharmaceutical composition, which comprises:
at least one of the compound, a stereoisomer, a pharmaceutically acceptable salt, or a pharmaceutically acceptable hydrate or solvate as described in the first aspect of the present application, and
a drug that needs to cross blood-brain barrier, which is selected from drug for treating disease or disorder of central nervous system, antidote to nerve agent, drug for treating glioma; and
one or more pharmaceutically acceptable carriers or excipients.

The eighth aspect of the present application provides a method for preventing and/or treating a human pathological condition or symptom, comprising administering to a patient in need thereof a prophylactically and/or therapeutically effective amount of the compound , a stereoisomer, a pharmaceutically acceptable salt, a pharmaceutically acceptable hydrate or solvate, or a pharmaceutically acceptable ester thereof as described in the first aspect of the present application, or the pharmaceutical composition as described in the third aspect of the present application, wherein the human pathological condition or symptom is related to the activity of A_{2A} adenosine receptor, and the prevention or treatment of the human pathological condition or symptom requires the activation of A_{2A} adenosine receptor.

The ninth aspect of the present application provides the compound represented by the general Formula (I), a stereoisomer, a pharmaceutically acceptable salt, a pharmaceutically acceptable hydrate or solvate, or a pharmaceutically acceptable ester thereof as described in the first aspect of the present application, for use in the prevention and/or treatment of a human pathological condition or symptom, wherein the human pathological condition or symptom is related to the activity of A_{2A} adenosine receptor, and the prevention or treatment of the human pathological or symptom requires the activation of A_{2A} adenosine receptor.

The tenth aspect of the present application provides the compound represented by the general Formula (I), a stereoisomer, a pharmaceutically acceptable salt, a pharmaceutically acceptable hydrate or solvate, or a pharmaceutically acceptable ester thereof as described in the first aspect of the present application, for use as an A_{2A} adenosine receptor agonist or coronary vasodilator, or
for use in diagnosing a human abnormal myocardial perfusion, or
for use in increasing the permeability of blood-brain barrier of a subject receiving a therapeutic drug, and the subject benefits from the increased permeability of blood-brain barrier for delivering the therapeutic drug across the blood-brain barrier,

Preferably, the therapeutic drug is selected from the group consisting of: drug for treating disease or disorder of central nervous system, antidote to nerve agent, and drug for treating glioma.

The eleventh aspect of the present application also provides a method for diagnosing human abnormal myocardial perfusion, comprising administering a patient in need thereof a diagnostically effective amount of the compound, a stereoisomer, a pharmaceutically acceptable salt, a pharmaceutically acceptable hydrate or solvate, or a pharmaceutically acceptable ester thereof as described in the first aspect of the present application, or the pharmaceutical composition as described in the third aspect of the present application.

The twelfth aspect of the present application also provides a method for increasing the permeability of blood-brain barrier of a subject receiving a therapeutic drug, wherein the method comprises administering to the subject an effective amount of the compound, a stereoisomer, a pharmaceutically acceptable salt, a pharmaceutically acceptable hydrate or solvate, or a pharmaceutically acceptable ester thereof as described in the first aspect of the present application, or the pharmaceutical composition as described in the third aspect of the present application, wherein the subject benefits from the increased permeability of blood-brain barrier for delivering the therapeutic drug cross the blood-brain barrier.

According to some embodiments of the present application, in the method described in the twelfth aspect of the present application, the therapeutic drug is selected from the group consisting of: drug for treating disease or disorder of central nervous system, antidote to nerve agent, and drug for treating glioma.

According to some embodiments of the present application, the human pathological condition or symptom described in the present application is selected from the group consisting of: autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, foundrymen's disease, glaucoma, ocular hypertension, spinal cord injury, myocardial infarction, and acute myocardial infarction.

According to some embodiments of the present application, n is 1, 2 or 3 in the general Formula (I).

According to some embodiments of the present application, n is 1 in the general Formula (I).

According to some embodiments of the present application, n is 2 in the general Formula (I).

According to some embodiments of the present application, n is 3 in the general Formula (I).

According to some embodiments of the present application, each R in the general Formula (I) is independently selected from the group consisting of hydrogen, fluorine, chlorine, bromine, iodine, cyano, benzyloxy, fluorobenzyloxy, C₁₋₄ alkyl, halogenated C₁₋₄ alkyl, C₁₋₄ alkoxy, hydroxyl, C₁₋₄ alkylamino, di(C₁₋₄ alkyl)amino, phenylamino, diphenylamino, -NHC(O)R¹⁰, phenyl, pyridyl, pyrrolidinyl, cyclopentyl, cyclohexyl, morpholinyl, imidazolyl, wherein R¹⁰ is C₁₋₄ alkyl.

According to some embodiments of the present application, each R in the general Formula (I) is independently hydrogen, di(C₁₋₆ alkyl)amino, C₁₋₆ alkylamino, benzyloxy, halogenated benzyloxy, phenyl, halophenyl or cyano.

According to some embodiments of the present application, each R in the general Formula (I) is independently hydrogen, di(C₁₋₄ alkyl)amino, C₁₋₄ alkylamino, benzyloxy, halogenated benzyloxy, phenyl, halophenyl or cyano.

According to some embodiments of the present application, each R in the general Formula (I) is independently hydrogen, benzyloxy, phenyl, 4-fluorobenzyl, diethylamino, or cyano.

According to some embodiments of the present application, each R in the general Formula (I) is independently di(C₁₋₆ alkyl)amino.

According to some embodiments of the present application, each R in the general Formula (I) is independently C₁₋₆ alkylamino.

According to some embodiments of the present application, each R in the general Formula (I) is independently selected from the group consisting of: hydrogen, fluorine, chlorine, bromine, iodine, cyano, benzyloxy, fluorobenzyloxy, methyl, ethyl, n-propyl, isopropyl, n-butyl, isobutyl, tert-butyl, n-pentyl, tert-pentyl, neopentyl, hexyl, trifluoromethyl, difluoromethyl, fluoromethyl, methoxy, ethoxy, n-propoxy, isopropoxy, n-butoxy, tert-butoxy, sec-butoxy, n-pentyloxy, n-hexyloxy, hydroxyl, methylamino, ethylamino, propylamino, butylamino, dimethylamino, diethylamino, dipropylamino, dibutylamino, phenylamino, diphenylamino, acetamido, formylamino, propionamido, phenyl, pyridyl, pyrrolidinyl, cyclopentyl, cyclohexyl, morpholinyl, and imidazolyl.

According to some embodiments of the present application, each R in the general Formula (I) is independently selected from the group consisting of: hydrogen, methoxy, ethoxy, acetamido, benzyloxy, trifluoromethyl, diphenylamino, 4-fluorobenzyloxy, chlorine, pyridin-2-yl, phenyl, pyrrolidin-1-yl, 1H-imidazol-1-yl, propoxy, diethylamino, hydroxyl, morpholin-4-yl, and cyano.

According to some embodiments of the present application, the compound represented by the general Formula (I) of the present application has the structure represented by the Formula (I-1), 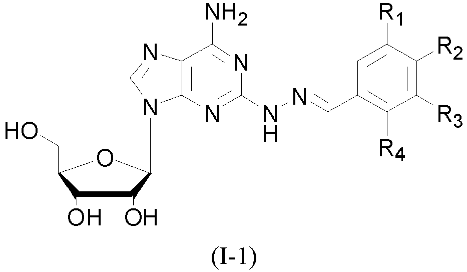 wherein, R₁, R₂, R₃, R₄ are each independently selected from the group consisting of: hydrogen, halogen, cyano, benzyloxy, halogenated benzyloxy, C₁₋₆ alkyl, halogenated C₁₋₆ alkyl, C₁₋₆ alkoxy, hydroxyl, C₁₋₆ alkylamino, di(C₁₋₆ alkyl)amino, anilino, diphenylamino, phenylamino,-NHC(O)R¹⁰, aryl, heteroaryl, cycloalkyl, and heterocycloalkyl, wherein R¹⁰ is C₁₋₆ alkyl.

According to some embodiments of the present application, R₁, R₂, R₃, and R₄ in the Formula (1-1) are each independently selected from the group consisting of: hydrogen, fluorine, chlorine, bromine, iodine, cyano, benzyloxy, fluorobenzyloxy, C₁₋₄ alkyl, halogenated C₁₋₄ alkyl, C₁₋₄ alkoxy, hydroxyl, C₁₋₄ alkylamino, di(C₁₋₄ alkyl)amino, phenylamino, diphenylamino,-NHC(O)R¹⁰, phenyl, pyridyl, pyrrolidinyl, cyclopentyl, cyclohexyl, morpholinyl, imidazolyl, wherein R¹⁰ is C₁₋₄ alkyl.

According to some embodiments of the present application, R₁, R₂, R₃, and R₄ in the Formula (1-1) are each independently selected from the group consisting of: hydrogen, fluorine, chlorine, bromine, iodine, cyano, benzyloxy, fluorobenzyloxy, methyl, ethyl, n-propyl, isopropyl, n-butyl, isobutyl, tert-butyl, n-pentyl, tert-pentyl, neopentyl, hexyl, trifluoromethyl, difluoromethyl, fluoromethyl, methoxy, ethoxy, n-propoxy, isopropoxy, n-butoxy, tert-butoxy, sec-butoxy, n-pentyloxy, n-hexyloxy, hydroxyl, methylamino, ethylamino, propylamino, butylamino, dimethylamino, diethylamino, dipropylamino, dibutylamino, phenylamino, diphenylamino, acetamido, formylamino, propionamido, phenyl, pyridyl, pyrrolidinyl, cyclopentyl, cyclohexyl, morpholinyl, and imidazolyl.

According to some embodiments of the present application, R₁, R₂, R₃, and R₄ in the Formula (1-1) are each independently selected from the group consisting of: hydrogen, methoxy, ethoxy, acetyl, acetamido, benzyloxy, trifluoromethyl, diphenylamino, 4-fluorobenzyloxy, chlorine, pyridin-2-yl, phenyl, pyrrolidin-1-yl, 1H-imidazol-1-yl, propoxy, diethylamino, hydroxyl, morpholin-4-yl, and cyano.

According to some embodiments of the present application, R₁ in the Formula (1-1) is hydrogen or methoxy.

According to some embodiments of the present application, R₁ in the Formula (1-1) is hydrogen.

According to some embodiments of the present application, R₂ in the Formula (1-1) is di(C₁₋₆ alkyl)amino.

According to some embodiments of the present application, R₂ in the Formula (1-1) is C₁₋₆ alkylamino.

According to some embodiments of the present application, R₂ in the Formula (1-1) is di(C₁₋₄ alkyl)amino.

According to some embodiments of the present application, R₂ in the Formula (1-1) is C₁₋₄ alkylamino.

According to some embodiments of the present application, R₂ in the Formula (1-1) is benzyloxy or halogenated benzyloxy,

According to some embodiments of the present application, R₂ in the Formula (I) is phenyl.

According to some embodiments of the present application, R₂ in the Formula (I) is halogenated phenyl.

According to some embodiments of the present application, R₂ in the Formula (1-1) is hydrogen, methoxy, acetyl, benzyloxy, trifluoromethyl, diphenylamino, 4-fluorobenzyloxy, chlorine, pyridin-2-yl, phenyl, pyrrolidin-1-yl, 1H-imidazol-1-yl, propoxy, diethylamino, hydroxyl, morpholin-4-yl, or cyano.

According to some embodiments of the present application, R₃ in the Formula (1-1) is hydrogen, benzyloxy, trifluoromethyl, ethoxy, or methoxy.

According to some embodiments of the present application, R₃ in the Formula (1-1) is hydrogen or benzyloxy.

According to some embodiments of the present application, R₃ in the Formula (1-1) is benzyloxy.

According to some embodiments of the present application, R₄ in the Formula (1-1) is hydrogen, trifluoromethyl or cyano.

According to some embodiments of the present application, R₄ in the Formula (1-1) is hydrogen.

According to some embodiments of the present application, R₁, R₃, and R₄ in the Formula (I-1) are each independently hydrogen; R₂ is di(C₁₋₆ alkyl)amino, C₁₋₆ alkylamino, benzyloxy, halogenated benzyloxy, phenyl, halogenated phenyl or cyano.

According to some embodiments of the present application, R₁ and R₄ in the Formula (1-1) are each independently hydrogen; R₂ is di(C₁₋₆ alkyl)amino, C₁₋₆ alkylamino, benzyloxy, halogenated benzyloxy, phenyl, halogenated phenyl or cyano; R₃ is hydrogen or benzyloxy.

According to some embodiments of the present application, in the Formula (1-1), R₁ and R₄ are each independently hydrogen; R₂ is benzyloxy, phenyl, 4-fluorobenzyl, diethylamino, or cyano; R₃ is hydrogen or benzyloxy.

According to some embodiments of the present application, the compound represented by the general Formula (I) described in the first aspect of the present application is selected from the group consisting of:
N-{4-{(E)-{2-{6-amino-9-[(2R,3R,4S,5R)-3,4-dihydroxy-5-(hydroxymethyl)tetrahydrofuran-2-yl]-9H-purin-2-yl}hydrazono}methyl}phenyl}acetamide;
(2R,3R,4S,5R)-2-{6-amino-2-{2-[(E)-3,4-bis(benzyloxy)benzylidene]hydrazino}-9H-pruin-9-yl}-5-(hydroxymethyl)tetrahydrofuran-3,4-diol;
(2R,3R,4S,5R)-2-{6-amino-2-{2-[(E)-2,4-bis(trifluoromethyl)benzylidene]hydrazino}-9H-pruin-9-yl}-5-(hydroxymethyl)tetrahydrofuran-3,4-diol;
(2R,3R,4S,5R)-2-{6-amino-2-{2-[(E)-4-(diphenylamino)benzylidene]hydrazino}-9H-pruin-9-yl}-5-(hydroxymethyl)tetrahydrofuran-3,4-diol;
(2R,3R,4S,5R)-2-{6-amino-2-{2-{(E)-4-[(4-fluorobenzyl)oxy]benzylidene}hydrazino}-9H-purin-9-yl}-5-(hydroxymethyl)tetrahydrofuran-3,4-diol;
(2R,3R,4S,5R)-2-{6-amino-2-{2-[(E)-3-(benzyloxy)benzylidene]hydrazino}-9H-purin-9-yl}-5-(hydroxymethyl)tetrahydrofuran-3,4-diol;
(2R,3R,4S,5R)-2-{6-amino-2-{2-[(E)-4-chloro-3-(trifluoromethyl)benzylidene]hydrazino}-9H-purin-9-yl}-5-(hydroxymethyl)tetrahydrofuran-3,4-diol;
(2R,3R,4S,5R)-2-{6-amino-2-{2-[(E)-4-(pyridin-2-yl)benzylidene]hydrazino)-9H-purin-9-yl}-5-(hydroxymethyl)tetrahydrofuran-3,4-diol;
(2R,3R,4S,5R)-2-{2-{2-[(E)-[1,1'-biphenyl]-4-yl-methylene]hydrazino}-6-amino-9H-purin-9-yl}-5-(hydroxymethyl)tetrahydrofuran-3,4-diol;
(2R,3R,4S,5R)-2-{6-amino-2-{2-[(E)-4-(pyrrolidin-1-yl)benzylidene]hydrazino)-9H-pruin-9-yl}-5-(hydroxymethyl)tetrahydrofuran-3,4-diol;
(2R,3R,4S,5R)-2-{6-amino-2-{2-[(E)-4-(trifluoromethyl)benzylidene]hydrazino}-9H-purin-9-yl}-5-(hydroxymethyl)tetrahydrofuran-3,4-diol;
(2R,3R,4S,5R)-2-{2-{2-[(E)-4-(1H-imidazol-1-yl)benzylidene]hydrazino}-6-amino-9H-pruin-9-yl}-5-(hydroxymethyl)tetrahydrofuran-3,4-diol;
(2R,3R,4S,5R)-2-{6-amino-2-{2-[(E)-4-propoxybenzylidene]hydrazino}-9H-purin-9-yl}-5-(hydroxymethyl)tetrahydrofuran-3,4-diol;
2-{(E)-{2-{6-amino-9-[(2R,3R,4S,5R)-3,4-dihydroxy-5-(hydroxymethyl)tetrahydrofuran-2-yl]-9H-purinylpyridin-2-yl}hydrazono}methyl}benzonitrile;
(2R,3R,4S,5R)-2-{6-amino-2-{2-[(E)-4-(diethylamino)benzylidene]hydrazino}-9H-purin-9-yl}-5-(hydroxymethyl)tetrahydrofuran-3,4-diol;
(2R,3R,4S,5R)-2-{6-amino-2-{2-[(E)-3-ethoxy-4-hydroxybenzylidene]hydrazino}-9H-purin-9-yl}-5-(hydroxymethyl)tetrahydrofuran-3,4-diol;
(2R,3R,4S,5R)-2-{6-amino-2-{2-[(E)-4-morpholinobenzylidene]hydrazino}-9H-purin-9-yl}-5-(hydroxymethyl)tetrahydrofuran-3,4-diol;
(2R,3R,4S,5R)-2-{6-amino-2-{2-[(E)-3,4,5-trimethoxybenzylidene]hydrazino}-9H-purin-9-yl}-5-(hydroxymethyl)tetrahydrofuran-3,4-diol;
(2R,3R,4S,5R)-2-{6-amino-2-{2-[(E)-4-(benzyloxy)-3-methoxybenzylidene]hydrazino}-9H-purin-9-yl}-5-(hydroxymethyl)tetrahydrofuran-3,4-diol;
4-{E-{2-{6-amino-9-[(2R,3R,4S,5R)-3,4-dihydroxy-5-(hydroxymethyl)tetrahydrofuran-2-yl]-9H-purinylpyridin-2-yl}hydrazono}methyl}benzonitrile.

According to some embodiments of the present application, the method described in the second aspect of the present application has a synthesis reaction process as follows:

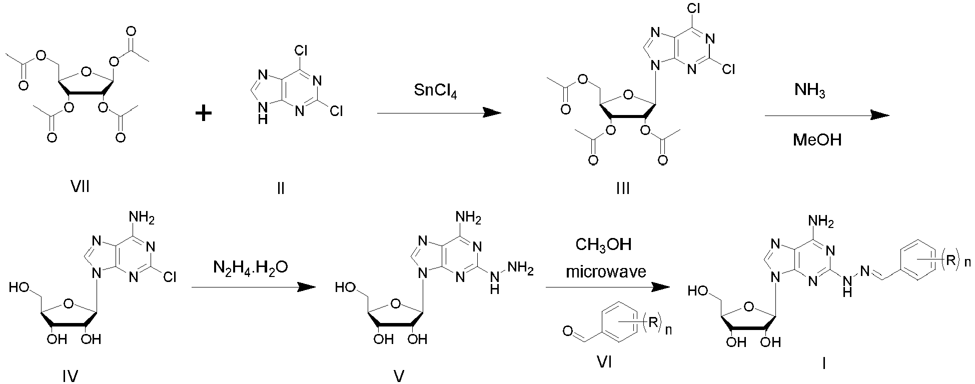

Ribofuranose tetraacetate (Compound of Formula VII) as starting material is subjected to a substitution reaction with 2,6-dichloropurine (Compound of formula II) in the presence of tin tetrachloride as catalyst at 110°C to 130°C to produce 2,6-dichloro-2',3',5'-triacetylpurine nucleoside (Compound of Formula III); the obtained 2,6-dichloro-2',3',5'-triacetylpurine nucleoside (Compound of formula III) is subjected to a ammonolysis in a method solution of ammonia under sealing condition to obtain 2-chloroadenosine (Compound of Formula IV); 2-chloroadenosine (Compound of Formula IV) is subjected to hydrazinolysis with hydrazine hydrate at 40°C to 60°C to produce 2-hydrazinoadenosine (Compound of Formula V); finally, 2-hydrazinoadenosine (Compound of Formula V) reacts with a substituted benzaldehyde (Compound of Formula VI) in a methanol solution at 70°C to 90°C under microwaves to obtain a 2-benzylidenehydrazinoadenosine compound (Compounds of Formula I), wherein the definitions of the substituent R and n are the same as those described in the first aspect of the present application, and can be selected as required.

### Definition of substituent

The term "alkyl" as used herein refers to a saturated linear or branched monovalent hydrocarbonyl preferably having 1 to 6, 1 to 4 or 1 to 3 carbon atoms. For example, "C₁₋₆ alkyl" refers to a saturated linear or branched monovalent hydrocarbonyl having 1 to 6 carbon atoms. Typical examples of "alkyl" include, but are not limited to, methyl, ethyl, n-propyl, isopropyl, n-butyl, isobutyl, tert-butyl, n-pentyl, tert-pentyl, neopentyl, hexyl.

The term "hydroxyl" as used herein refers to -OH.

The term "halogen" as used herein refers to fluorine, chlorine, bromine or iodine. The preferred halogen group is fluorine, chlorine or bromine.

The term "halogenated C₁₋₆ alkyl" as used herein refers to C₁₋₆ alkyl mono- or polysubstituted by halogen such as fluorine, chlorine, bromine or iodine. The preferred halogenated alkyl groups include chloromethyl, chloroethyl, dichloroethyl, trifluoromethyl, difluoromethyl, monofluoromethyl and the like.

The term "C₁₋₆ alkylamino" as used herein refers to an amino group substituted with one C₁₋₆ alkyl. Typical examples of "C₁₋₆ alkylamino" include but are not limited to methylamino, ethylamino, propylamino, butylamino and the like.

The term "di(C₁₋₆ alkyl)amino" as used herein refers to an amino group substituted with two C₁₋₆ alkyl groups. Typical examples of "di(C₁₋₆ alkyl)amino" include, but are not limited to, dimethylamino, diethylamino, dipropylamino, dibutylamino and the like.

The term "cycloalkyl" as used herein refers to a saturated cyclic hydrocarbonyl having 3 to 12 carbon atoms and having monocyclic or bicyclic or multiple rings (including fused and bridged ring systems), preferably having 3 to 10, 3 to 8, 5 to 8, 3 to 6 or 5 to 6 carbon atoms. Typical examples of "cycloalkyl" include, but are not limited to, monocyclic structures such as cyclopropyl, cyclobutyl, cyclopentyl, cyclohexyl, cycloheptyl, cyclooctyl, and so on, bicyclic structures such as bicyclo[2.2.1]heptyl, and polycyclic structures such as adamantyl, etc.

The term "heterocycloalkyl" as used herein refers to a cycloalkyl as defined herein containing one, two or more heteroatoms independently selected from N, O and S. Typical examples of "heterocycloalkyl" include, but are not limited to, tetrahydrofuranyl, tetrahydrothienyl, pyrrolidinyl, piperazinyl, thiazinyl, piperidinyl, morpholinyl and the like.

The term "aryl" as used herein refers to an unsaturated aromatic carbocyclic group having 5 to 14 carbon atoms and having a monocyclic ring or fused ring of two or more rings. The aryl preferably has 5 to 10, 5 to 8 or 5 to 6 carbon atoms. Typical examples of "aryl" include, but are not limited to, phenyl, naphthyl, anthryl and the like.

The term "heteroaryl" as used herein refers to a heteroaromatic cyclic group having 5 to 14 ring members, including monocyclic heteroaromatic ring and polycyclic aromatic ring, in which the monocyclic aromatic ring is fused with one or more other aromatic rings. The heteroaryl has one or two or more heteroatoms selected from O, S or N. The term "heteroaryl" as used herein also includes groups in which an aromatic ring is fused with one or more non-aromatic (carbocyclic or heterocyclic) rings, wherein the linking group or point is located on the aromatic ring or non-aromatic ring. The heteroaryl preferably has 5 to 10 ring members, more preferably 5 to 6 ring members. Typical examples of "heteroaryl" include, but are not limited to, furyl, imidazolyl, triazolyl, indolyl, tetrazolyl, pyridyl, pteridyl, pyrimidinyl, triazolyl, quinolinyl, isoquinolinyl, quinazolinyl, quinoxalinyl and the like.

The term "C₁₋₆ alkoxy" as used herein refers to -OR¹¹, wherein R¹¹ is C₁₋₆ alkyl as defined herein. Typical examples of "C₁₋₆ alkoxy" include, but are not limited to, methoxy, ethoxy, n-propoxy, isopropoxy, n-butoxy, tert-butoxy, sec-butoxy, n-pentyloxy, n-hexyloxy, 1,2-dimethylbutoxy, etc.

When the name of compound used herein is inconsistent with the chemical structural formula, the chemical structural formula shall prevail.

According to some embodiments of the present application, the pharmaceutically acceptable salt of the compound of general Formula (I) described in the present application includes salts formed with inorganic or organic acids, and salts formed with inorganic or organic bases. The present application relates to all forms of the above-mentioned salts, includes but not limited to: sodium salt, potassium salt, calcium salt, lithium salt, meglumine salt, hydrochloride, hydrobromide, hydriodate, nitrate, sulfate, hydrogen sulfate, phosphate, hydrogen phosphate, acetate, propionate, butyrate, oxalate, trimethylacetate, adipate, alginate, lactate, citrate, tartrate, succinate, maleate, fumarate, picrate, aspartate, gluconate, benzoate, methanesulfonate, ethanesulfonate, benzenesulfonate, p-toluenesulfonate and pamoate.

According to some embodiments of the present application, the compound of general Formula (I) described in the present application can form a pharmaceutically acceptable ester with an organic or inorganic acid. The pharmaceutically acceptable ester includes phosphate, sulfate, nitrate, formate, acetate, propionate, butyrate, valerate, and caproate, which are hydrolyzable in vivo.

The carrier described in the present application includes, but is not limited to: ion exchanger, alumina, aluminum stearate, lecithin, serum protein such as human albumin, buffer substance such as phosphate, glycerol, sorbic acid, potassium sorbate, mixture of partial glyceride of saturated plant fatty acid, water, salt or electrolyte such as protamine sulfate, disodium hydrogen phosphate, potassium hydrogen phosphate, sodium chloride, zinc salt, colloidal silica, magnesium trisilicate, polyvinylpyrrolidone, cellulosic substance, polyethylene glycol, sodium carboxymethylcellulose, polyacrylate, beeswax, lanolin.

The term "excipient" as used in the present application refers to an additive other than the main active ingredient in a pharmaceutical preparation. It is stable in nature, has no incompatibility with the main active ingredient, does not produce side effects, does not affect therapeutic effect, is not prone to deform, dry, crack, mildew, be worm-eaten at room temperature, is harmless to the human body, has no physiological effect, does not produce chemical or physical effects on the function of main active ingredient, does not affect the content determination of the main active ingredient, etc. For example, binder, filler, disintegrant, lubricant in tablet; preservative, antioxidant, corrigent, flavoring agent, cosolvent, emulsifier, solubilizer, osmotic pressure regulator and coloring agent in oral liquid preparation, etc. can all be called excipients.

The pharmaceutical composition described in the present application can be administered through various routes, such as oral tablet, capsule, powder, oral liquid, injection and transdermal preparation. The above-mentioned various preparation forms can be prepared according to conventional methods in the field of pharmacy. According to conventional pharmaceutical practices, the pharmaceutically acceptable carrier includes diluent, filler, disintegrant, wetting agent, lubricant, coloring agent, flavoring agent or other conventional additives. Typical pharmaceutically acceptable carriers include, for example, microcrystalline cellulose, starch, crospovidone, povidone, polyvinylpyrrolidone, maltitol, citric acid, sodium laurylsulfonate or magnesium stearate, etc.

According to the present application, the pharmaceutical composition can be administered in any of the following routes: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or administration with the aid of an explanted reservoir.

As described herein, "effective amount" refers to an amount that is sufficient to treat or prevent or diagnose a patient's disease but is sufficiently low to avoid serious side effects (at a reasonable benefit/risk ratio) within the scope of reasonable medical judgment. The therapeutically or prophylactically or diagnostically effective amount of the compound will vary according on the factors such as the specific compound selected (for example, considering the potency, effectiveness and half-life of the compound), the route of administration selected, the disease to be treated or prevented or diagnosed, the severity of the disease to be treated or prevented or diagnosed, the age, size, weight and physical disease of the patient being treated, the medical history of the patient to be treated, the duration of treatment or prevention or diagnosis, the nature of concurrent therapy, the desired effects of the treatment or prevention or diagnosis and so on, but it can still be routinely determined by those skilled in the art.

In addition, it should be noted that the specific dosage and usage of the compound of general Formula (I) described in the present application for different patients depends on many factors, including the patient's age, weight, gender, natural health status, nutritional status, and the active strength of the compound, the administration time, metabolic rate, severity of disease and the subjective judgment of physician. Herein it is preferable to use a dose of 0.001 to 1000 mg/kg body weight/day.

### Beneficial technical effects of the present application

The compound represented by general Formula (I), a stereoisomer, a pharmaceutically acceptable salt or hydrate as provided in the present application can agonize A_{2A} adenosine receptor, so that it can be used to prevent or treat a human pathological condition or symptom, in which the human pathological condition or symptom can be improved by agonizing the activity of A_{2A} adenosine receptor.

### Specific Models for Carrying Out the Present Application

The present application can be further described through the following examples and test examples. However, the scope of the present application is not limited to the following examples or test examples. Those skilled in the art can understand that various changes and modifications can be made to the present application without departing from the spirit and scope of the present application. The present application provides a general and/or specific description of the materials and test methods used in the test. Although many materials and operating methods used to achieve the purpose of the present application are well known in the art, the present application is still described here in as much detail as possible.

For all the following examples, standard operations and purification methods known to those skilled in the art could be used. Unless otherwise stated, all temperatures were expressed in °C (Celsius). The structure of the compound was determined by nuclear magnetic resonance (NMR) or mass spectrometry (MS). The compound's melting point m.p. was determined by RY-1 melting point meter, in which the thermometer had not been corrected, and the m.p. was given in °C. 1H NMR was measured by JEOL JNM-ECA-400 NMR spectrometer. The mass spectrum was measured by API3000 (ESI) instrument. All solvents in reaction that were not specified were subject to standardized pretreatment.

### Example 1: Synthesis ofN-{4-{(E)-{2-{6-amino-9-[(2R,3R,4S,5R)-3,4-dihydroxy-5-(hydroxymethyl)tetrahydrofuran-2-yl]-9H-purin-2-yl}hydrazono}methyl}phenyl}acetamide (Compound 1)

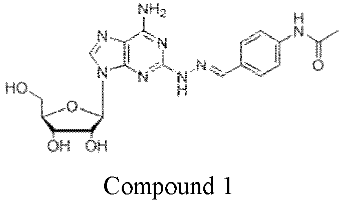

### 1.1 Synthesis of (2R,3R,4R,5R)-2-(acetoxymethyl)-5-(2,6-dichloro-9H-purin-9-yl) tetrahydrofuran-3,4-diyl diacetate (Compound of formula III)

21g (0.066mol) of (2S,3R,4R,5R)-5-(acetoxymethyl)tetrahydrofuran-2,3,4-triyl triacetate (Compound of Formula VII) was heated to 90°C until it became clear, and then 12g (0.063mol) of 2,6-dichloropurine (Compound of Formula II) and 0.3g of tin tetrachloride were added and stirred. The reaction solution was further heated to 120°C and stirred for 15 minutes. Then the solvent was evaporated in vacuum and the residue was cooled. Methanol (50 ml) was added to the residue, and the crude solid product was separated by filtration. The crude product was recrystallized in ethanol to obtain 12g of pale yellow powder product (2R,3R,4R,5R)-2-(acetoxymethyl)-5-(2,6-dichloro-9H-purin-9-yl)tetrahydrofuran-3,4- diyl diacetate (Compound of Formula III), which was directly used in the next reaction.

### 1.2 Synthesis of (2R,3R,4S,5R)-2-(6-amino-2-chloro-9H-purin-9-yl)-5-(hydroxymethyl) tetrahydrofuran-3,4-diol (Compound of Formula IV)

10g (0.022mol) of (2R,3R,4R,5R)-2-(acetoxymethyl)-5-(2,6-dichloro-9H-purin-9-yl) tetrahydrofuran-3,4-diyl diacetate (Compound of Formula III) was heated to 100°C in 200ml of solution of ammonia in methanol and kept for 24 hours in an autoclave. The solution was further stirred for 24 hours to room temperature, and then the solution was evaporated to dryness under reduced pressure to remove ammonia. The residue was purified by flash chromatography, in which a mixed solvent of CH₂Cl₂ and MeOH (CH₂Cl₂:MeOH = 10:1 (v/v)) was used as the eluent, and the product was dried below 50°C to obtain 4.5g of light yellow powder (2R,3R,4S,5R)-2-(6-amino-2-chloro-9H-purin-9-yl)-5-(hydroxymethyl)tetrahydrofuran-3,4-diol (Compound of Formula IV), which was directly used in the next reaction.

### 1.3 Synthesis of (2R,3R,4S,5R)-2-(6-amino-2-hydrazino-9H-purin-9-yl)-5-(hydroxymethyl) tetrahydrofuran-3,4-diol (Compound of Formula V)

5g (0.017mol) of (2R,3R,4S,5R)-2-(6-amino-2-chloro-9H-purin-9-yl)-5-(hydroxymethyl)tetrahydrofuran-3,4-diol (Compound of Formula IV) was added to 25ml of hydrazine hydrate (65wt% aqueous solution), the resulting mixture was heated to 50°C while stirring, continuously heated for 4 hours until the reactant (Compound of Formula IV) disappeared, in which the progress of the reaction was monitored by TLC (CH₂Cl₂:MeOH = 3:1 (v/v)). Then the reaction mixture was heated to 25°C, and 2-propanol (50ml) was added for dilution, then stirred overnight. The separated precipitate was filtered to obtain 4.4g of yellow solid (2R,3R,4S,5R)-2-(6-amino-2-hydrazino-9H-purin-9-yl)- 5-(hydroxymethyl)tetrahydrofuran-3,4-diol (Compound of Formula V), which was directly used in the next reaction.

### 1.4 Synthesis of N-{4-{(E)-{2-{6-amino-9-[(2R,3R,4S,5R)-3,4-dihydroxy-5-(hydroxymethyl)tetrahydrofuran-2-yl]-9H-purin-2-yl}hydrazono}methyl}phenyl}acetamide (Compound 1)

0.5g (0.0017mol) of (2R,3R,4S,5R)-2-(6-amino-2-hydrazino-9H-purin-9-yl)- 5-(hydroxymethyl)tetrahydrofuran-3,4-diol (Compound of Formula V) and 0.31g (0.0019mol) of 4-acetamidobenzaldehyde (1.1 equivalent) were mixed in methanol (30ml) and heated by microwave at 80°C for 30 minutes. The crude product was precipitated from methanol. After filtration, the crude product was further purified on a C18 reverse phase column using preparative medium pressure chromatography to obtain 171 mg of white solid (Compound 1). m.p. 126°C; ¹H NMR (DMSO-*d₆*): δ (ppm) 10.54 (s, 1H), 10.03 (s, 1H), 8.02 (s, 2H), 7.66-7.59 (m, 4H), 7.03 (br, 2H), 5.79 (d, 1H, J=6.4Hz), 5.44 (d, 1H, J=6.0Hz), 5.24-5.21 (m, 1H), 5.11 (d, 1H, J=4.4), 4.67 (dd, 1H, J=5.6Hz,5.6Hz), 4.24-4.21 (m, 1H), 3.98-3.96 (m, 1H), 3.75-3.57 (m, 2H), 2.06 (s, 3H); HRMS (ESI+) m/z [M+H]⁺ calculated for C₁₉H₂₂N₈O₅: 443.1786; found: 443.1786.

Compounds 2 to 20 could be prepared by referring to the method of Example 1, using different reactants (various substituted benzaldehydes represented by Formula VI) instead of 4-acetamidobenzaldehyde in step 1.4.

### Example 2: Synthesis of (2R,3R,4S,5R)-2-{6-amino-2-{2-[(E)-3,4-bis(benzyloxy) benzylidene]hydrazino}-9H-purin-9-yl}-5-(hydroxymethyl)tetrahydrofuran-3,4-diol (Compound 2)

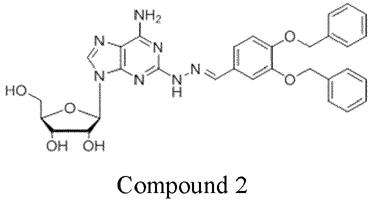

By referring to the method of step 1.4 in Example 1, and replacing 4-acetamidobenzaldehyde with 3,4-dibenzyloxybenzaldehyde, the title compound was obtained as 553 mg of white solid (Compound 2). m.p. 220°C; ¹H NMR (DMSO-*d₆*): δ (ppm) 10.55 (s, 1H), 8.00 (s, 1H), 7.98 (s, 1H), 7.96 (s, 1H), 7.52-7.30 (m, 10H), 7.09-7.05 (m, 4H), 5.76 (d, 1H, J=7.2Hz), 5.49-5.47 (m, 2H), 5.19-5.14 (m, 5H), 4.89-4.84 (m, 1H), 4.20-4.18 (m, 1H), 4.00 (s, 1H), 3.76-3.54 (m, 2H); HRMS (ESI+) m/z [M + H]⁺ calculated for C₃₁H₃₁N₇O₆: 598.2409; found: 598.2408.

### Example 3: Synthesis of (2R,3R,4S,5R)-2-{6-amino-2-{2-[(E)-2,4-bis(trifluoromethyl) benzylidene]hydrazino}-9H-purin-9-yl}-5-(hydroxymethyl)tetrahydrofuran-3,4-diol (Compound 3)

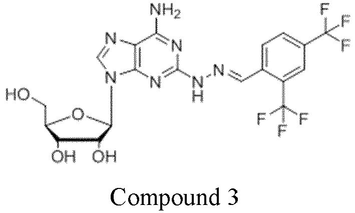

By referring to the method of step 1.4 in Example 1, and replacing 4-acetamidobenzaldehyde with 2,4-bis(trifluoromethyl)benzaldehyde, the title compound was obtained as 474 mg of white solid (Compound 3). m.p. 239°C; ¹H NMR (DMSO-*d₆*): δ (ppm) 11.44 (s, 1H), 8.70 (d, 1H, J=8.4Hz),8.47 (s, 1H), 8.12-8.01 (m, 3H), 7.31 (br, 2H), 5.81 (d, 1H, J=6.4Hz), 5.47 (br, 1H), 5.30 (br, 1H), 5.19 (br, 1H), 4.73-4.71 (m, 1H), 4.25-4.24 (m, 1H), 4.00-3.98 (m, 1H), 3.77-3.62 (m, 2H); HRMS (ESI+) m/z [M + H]⁺ calculated for C₁₉H₁₇F₆N₇O₄: 522.1319; found: 522.1319.

### Example 4: Synthesis of (2R,3R,4S,5R)-2-{6-amino-2-{2-[(E)-4-(diphenylamino) benzylidene]hydrazino}-9H-purin-9-yl}-5-(hydroxymethyl)tetrahydrofuran-3,4-diol (Compound 4)

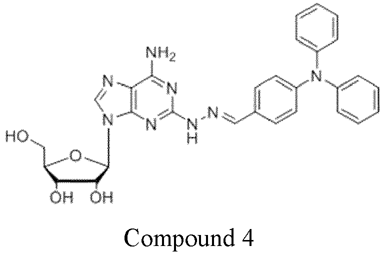

By referring to the method of step 1.4 in Example 1, and replacing 4-acetamidobenzaldehyde with 4-(N,N-diphenylamino)benzaldehyde, the title compound was obtained as 390 mg of yellow solid (Compound 4). m.p. 184°C; ¹H NMR (DMSO-*d₆*): δ (ppm) 10.56 (s, 1H), 8.01 (s, 2H), 7.63 (d, 2H,J=8.4Hz), 7.35-6.94 (m, 14H), 5.77 (d, 1H,J=6.4Hz), 5.45 (d, 1H,J=6.0Hz), 5.27-5.24 (m, 1H), 5.11 (d, 1H,J=4.4Hz), 4.70-4.66 (m, 1H), 4.21-4.18 (m, 1H), 3.93 (d, 1H,J=2.4Hz), 3.73-3.53 (m, 2H); HRMS (ESI+) m/z [M + H]⁺ calculated for C₂₉H₂₈N₈O₄: 553.2306; found: 553.2306.

### Example 5: Synthesis of (2R,3R,4S,5R)-2-{6-amino-2-{2-{(E)-4-[(4-fluorobenzyl)oxy] benzylidene}hydrazino}-9H-purin-9-yl}-5-(hydroxymethyl)tetrahydrofuran-3,4-diol (Compound 5)

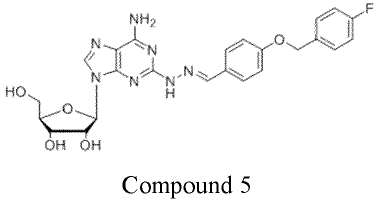

By referring to the method of step 1.4 in Example 1, and replacing 4-acetamidobenzaldehyde with 4-(4-fluorobenzyloxy)benzaldehyde, the title compound was obtained as 291 mg of white solid (Compound 5). m.p. 144°C; ¹H NMR (DMSO-*d₆*): δ (ppm) 10.51 (s, 1H), 8.02 (s, 2H), 7.68 (d, 2H, J=8.8Hz), 7.54-7.50 (m, 2H), 7.26-7.22 (m, 2H), 7.04 (d, 4H, J=8.8Hz), 5.80 (d, 1H, J=6.8Hz), 5.47 (d, 1H, J=6.4Hz), 5.31-5.28 (m, 1H), 5.16 (d, 1H, J=4.0Hz), 5.12 (s, 2H), 4.70-4.66 (m, 1H), 4.22-4.19 (m, 1H), 3.98-3.96 (m, 1H), 3.74-3.57 (m, 2H); HRMS (ESI+) m/z [M + H]⁺ calculated for C₂₄H₂₄FN₇O₅: 510.1896; found: 510.1895.

### Example 6: Synthesis of (2R,3R,4S,5R)-2-{6-amino-2-{2-[(E)-3-(benzyloxy)benzylidene] hydrazino}-9H-purin-9-yl}-5-(hydroxymethyl)tetrahydrofuran-3,4-diol (Compound 6)

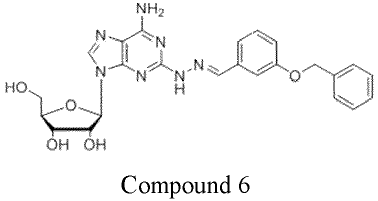

By referring to the method of step 1.4 in Example 1, and replacing 4-acetamidobenzaldehyde with 3-benzyloxybenzaldehyde, the title compound was obtained as 355 mg of white solid (Compound 6). m.p. 148°C; ¹H NMR (DMSO-*d₆*): δ (ppm) 10.73 (s, 1H), 8.05 (s, 2H), 7.62-6.95 (m, 11H), 5.79 (d, 1H, J=6.8Hz), 5.48 (d, 1H, J=6.0Hz), 5.38 (s, 1H), 5.16 (s, 3H), 4.79-4.75 (m, 1H), 4.19 (s, 1H), 3.98 (s, 1H), 3.74-3.55 (m, 2H); HRMS (ESI+) m/z [M + H]⁺ calculated for C₂₄H₂₅N₇O₅: 492.1990; found: 492.1990.

### Example 7: Synthesis of (2R,3R,4S,5R)-2-{6-amino-2-{2-[(E)-4-chloro-3-(trifluoromethyl) benzylidene]hydrazino}-9H-purin-9-yl}-5-(hydroxymethyl)tetrahydrofuran-3,4-diol (Compound 7)

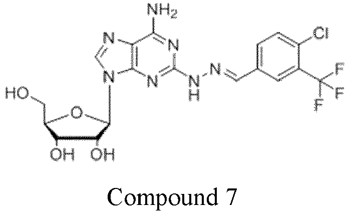

By referring to the method of step 1.4 in Example 1, and replacing 4-acetamidobenzaldehyde with 4-chloro-3-(trifluoromethyl)benzaldehyde, the title compound was obtained as 443 mg of white solid (Compound 7). m.p. 242°C; ¹H NMR (DMSO-*d₆*): δ (ppm) 10.94 (s, 1H), 8.46 (s, 1H), 8.07 (s, 1H), 8.01 (s, 1H), 7.89 (d, 1H, J=8.0Hz), 7.68 (d, 1H, J=8.4Hz), 7.13 (br, 2H), 5.73 (d, 1H, J=7.2Hz), 5.46 (d, 1H, J=5.2Hz), 5.39 (d, 1H, J=6.4Hz), 5.14 (s, 1H), 4.79-4.77 (m, 1H), 4.12 (s, 1H), 3.96 (s, 1H), 3.68-3.51 (m, 2H); HRMS (ESI+) m/z [M + H]⁺ calculated for C₁₈H₁₇ClF₃N₇O₄: 488.1055; found: 488.1055.

### Example 8: Synthesis of (2R,3R,4S,5R)-2-{6-amino-2-{2-[(E)-4-(pyridin-2-yl)benzylidene] hydrazino}-9H-purin-9-yl}-5-(hydroxymethyl)tetrahydrofuran-3,4-diol (Compound 8)

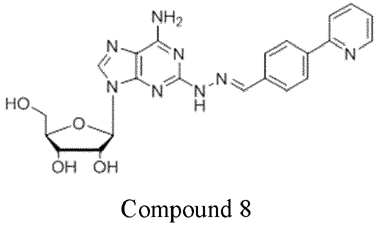

By referring to the method of step 1.4 in Example 1, and replacing 4-acetamidobenzaldehyde with 4-(2-pyridyl)benzaldehyde, the title compound was obtained as 537 mg of white solid (Compound 8). m.p. 164°C; ¹H NMR (DMSO-*d₆*): δ (ppm) 10.83 (s, 1H), 8.69 (s, 1H), 8.15-7.85 (m, 8H), 7.36-7.35 (m, 1H), 7.18 (br, 2H), 5.83 (d, 1H, J=6.4Hz), 5.50 (s, 1H), 5.28 (s, 1H), 5.20 (s, 1H), 4.71 (s, 1H), 4.25 (s, 1H), 4.00 (s, 1H), 3.77-3.62 (m, 2H); HRMS (ESI+) m/z [M + H]⁺ calculated for C₂₂H₂₂N₈O₄: 463.1837; found: 463.1838.

### Example 9: Synthesis of (2R,3R,4S,5R)-2-{2-{2-[(E)-[1,1'-biphenyl]-4-ylmethylene] hydrazino}-6-amino-9H-purin-9-yl}-5-(hydroxymethyl)tetrahydrofuran-3,4-diol (Compound 9)

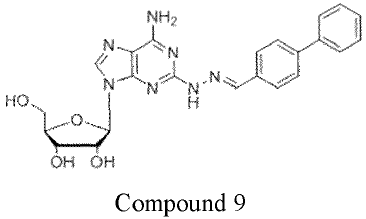

By referring to the method of step 1.4 in Example 1, and replacing 4-acetamidobenzaldehyde with 4-biphenyldehyde, the title compound was obtained as 467 mg of white solid (Compound 9). m.p. 234°C; ¹H NMR (DMSO-*d₆*): δ (ppm) 10.78 (s, 1H), 8.12 (s, 1H),8.07 (s, 1H), 7.83 (d, 2H, J=8.4Hz), 7.31-7.71 (m, 4H), 7.48 (t, 2H, J=7.2Hz), 7.38 (t, J=7.2Hz,1H), 7.16 (br, 2H), 5.82 (d, 1H, J=6.8Hz), 5.49 (d, 1H, J=6.0Hz), 5.28 (s, 1H), 5.19 (d, 1H, J=4.4Hz), 4.69-4.67 (m, 1H), 4.22 (d, 1H, J=2.4Hz), 3.98 (d, 1H, J=2.4Hz), 3.75-3.60 (m, 2H); HRMS (ESI+) m/z [M + H]⁺ calculated for C₂₃H₂₃N₇O₄: 462.1884; found: 462.1884.

### Example 10: Synthesis of (2R,3R,4S,5R)-2-{6-amino-2-{2-[(E)-4-(pyrrolidin-1-yl) benzylidene]hydrazino}-9H-purin-9-yl}-5-(hydroxymethyl)tetrahydrofuran-3,4-diol (Compound 10)

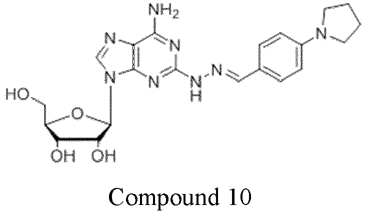

By referring to the method of step 1.4 in Example 1, and replacing 4-acetamidobenzaldehyde with 4-(1-pyrrolidinyl)benzaldehyde, the title compound was obtain as 352 mg of white solid (Compound 10). m.p. 174°C; ¹H NMR (DMSO-*d₆*): δ (ppm) 10.23 (s, 1H), 7.98 (s, 1H), 7.94 (s, 1H), 7.54 (d, 2H, J=8.8Hz), 6.95 (br, 2H), 6.54 (d, 2H, J=8.8Hz), 5.78 (d, 1H, J=6.8Hz), 5.44 (d, 1H, J=6.4Hz), 5.27-5.24 (m, 1H), 5.11 (d, 1H, J=4.0Hz), 4.69-4.65 (m, 1H), 4.20 (s, 1H), 3.97 (s, 1H), 3.73-3.57 (m, 2H), 3.26 (s, 4H), 1.96 (s, 4H); HRMS (ESI+) m/z [M + H]⁺ calculated for C₂₁H₂₆N₈O₄: 455.2150; found: 455.2150.

### Example 11: Synthesis of (2R,3R,4S,5R)-2-{6-amino-2-{2-[(E)-4-(trifluoromethyl) benzylidene]hydrazino}-9H-purin-9-yl)-5-(hydroxymethyl)tetrahydrofuran-3,4-diol (Compound 11)

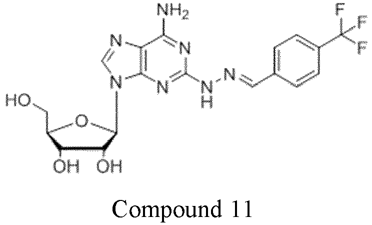

By referring to the method of step 1.4 in Example 1, and replacing 4-acetamidobenzaldehyde with 4-(trifluoromethyl)benzaldehyde, the title compound was obtained as 404 mg of white solid (Compound 11). m.p. 260°C; ¹H NMR (DMSO-*d₆*): δ (ppm) 10.97 (s, 1H), 8.14 (s, 1H), 8.08 (s, 1H), 7.96 (d, 2H, J=8.4Hz), 7.74 (d, 2H, J=8.4Hz), 7.18 (br, 2H), 5.82 (d, 1H, J=6.4Hz), 5.47 (d, 1H, J=5.6Hz), 5.29 (s, 1H), 5.18 (d, 1H, J=3.2Hz), 4.71-4.69 (m, 1H), 4.23 (s, 1H), 3.98 (d, 1H, J=2Hz), 3.76-3.60 (m, 2H); HRMS (ESI+) m/z [M + H]⁺ calculated for C₁₈H₁₈F₃N₇O₄: 454.1445; found: 454.1446.

### Example 12: Synthesis of (2R,3R,4S,5R)-2-{2-{2-[(E)-4-(1H-imidazol-1-yl)benzylidene] hydrazino}-6-amino-9H-purin-9-yl}-5-(hydroxymethyl)tetrahydrofuran-3,4-diol (Compound 12)

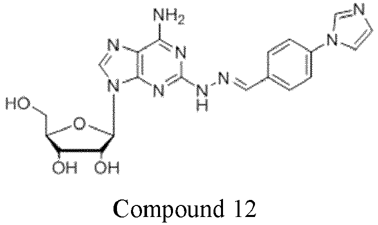

By referring to the method of step 1.4 in Example 1, and replacing 4-acetamidobenzaldehyde with 4-(1H-imidazol-1-yl)benzaldehyde, the title compound was obtained as 615 mg of white solid (Compound 12). m.p. 276°C; ¹H NMR (DMSO-*d₆*): δ (ppm) 10.77 (s, 1H), 8.33 (s, 1H), 8.11 (s, 1H), 8.05 (s, 1H), 7.89 (d, 2H, J=8.4Hz), 7.81 (s, 1H), 7.70 (d, 2H, J=8.8Hz), 7.13 (s, 1H), 7.11 (br, 2H), 5.81 (d, 1H, J=6.8Hz), 5.48 (d, 1H, J=6.4Hz), 5.35-5.32 (m, 1H), 5.19 (d, 1H, J=4.0Hz), 4.74-4.70 (m, 1H), 4.22-4.20 (m, 1H), 3.99 (s, 1H), 3.76-3.59 (m, 1H); HRMS (ESI+) m/z [M + H]⁺ calculated for C₂₀H₂₁N₉O₄: 452.1789; found: 452.1789.

### Example 13: Synthesis of (2R,3R,4S,5R)-2-{6-amino-2-{2-[(E)-4-propoxybenzylidene] hydrazino}-9H-purine-9-yl}-5-(hydroxymethyl)tetrahydrofuran-3,4-diol (Compound 13)

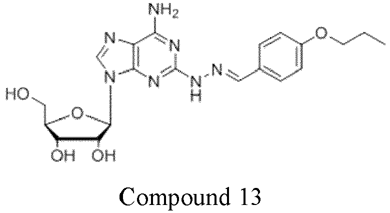

By referring to the method of step 1.4 in Example 1, and replacing 4-acetamidobenzaldehyde with 4-propoxybenzaldehyde, the title compound was obtained as 306 mg of white solid (Compound 13). m.p. 218°C; ¹H NMR (DMSO-*d₆*): δ (ppm) 10.55 (s, 1H), 8.04 (s, 1H), 8.02 (s, 1H), 7.67 (d, 2H, J=8.4Hz), 7.11 (br, 2H), 6.95 (d, 1H, J=8.8Hz), 5.80 (d, 1H, J=6.4Hz), 5.47 (d, 1H, J=5.2Hz), 5.28 (s, 1H), 5.17 (s, 1H), 4.68 (s, 1H), 4.20 (s, 1H), 3.97-3.94 (m, 3H), 3.73-3.61 (m, 2H), 1.76-1.71 (m, 2H), 0.98 (t, 3H, J=7.6Hz); HRMS (ESI+) m/z [M + H]⁺ calculated for C₂₀H₂₅N₇O₅: 444.1990; found: 444.1990.

### Example 14: Synthesis of 2-{(E)-{2-{6-amino-9-[(2R,3R,4S,5R)-3,4-dihydroxy-5-(hydroxymethyl)tetrahydrofuran-2-yl]-9H-purinylpyridin-2-yl}hydrazono}methyl}benzonitrile (Compound 14)

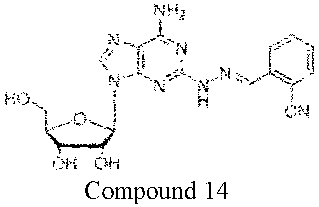

By referring to the method of step 1.4 in Example 1, and replacing 4-acetamidobenzaldehyde with 2-cyanobenzaldehyde, the title compound was obtained as 359mg of white solid (Compound 14). m.p. 260°C; ¹H NMR (DMSO-*d₆*): δ (ppm) 11.21 (s, 1H), 8.45 (s, 1H), 8.35 (d, 1H, J=8.0Hz), 8.08 (s, 1H), 7.83 (d, 1H, J=7.6Hz), 7.72 (t, 1H, J=7.6Hz), 7.48 (t, 1H, J=7.6Hz), 7.18 (br, 2H), 5.81 (d, 1H, J=6.4), 5.47 (d, 1H, J=5.6Hz), 5.29-5.27 (m, 1H), 5.17 (d, 1H, J=3.6Hz), 4.73-4.71 (m, 1H), 4.23 (s, 1H), 3.98 (s, 1H), 3.75-3.58 (m, 2H); HRMS (ESI+) m/z [M + H]⁺ calculated for C₁₈H₁₈N₈O₄: 411.1524; found: 411.1523.

### Example 15: Synthesis of (2R,3R,4S,5R)-2-{6-amino-2-{2-[(E)-4-(diethylamino) benzylidene]hydrazino}-9H-purin-9-yl}-5-(hydroxymethyl)tetrahydrofuran-3,4-diol (Compound 15)

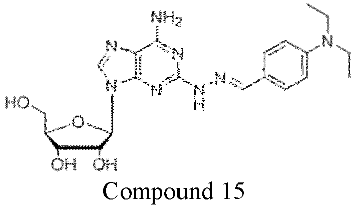

By referring to the method of step 1.4 in Example 1, and replacing 4-acetamidobenzaldehyde with 4-(N,N-diethyl)aminobenzaldehyde, the title compound was obtained as 299 mg of white solid (Compound 15). m.p. 164°C; ¹H NMR (DMSO-*d₆*): δ (ppm) 10.27 (s, 1H), 7.99 (s, 1H), 7.92 (s, 1H), 7.51 (d, 2H, J=8.8Hz), 6.99 (br, 2H), 6.66 (d, 2H, J=8.8Hz), 5.78 (d, 1H, J=6.8Hz), 5.47 (d, 1H, J=6Hz), 5.30-5.26 (m, 1H), 5.15 (d, 1H, J=4.4Hz), 4.69-4.65 (m, 1H), 4.21-4.18 (m, 1H), 3.97-3.95 (m, 1H), 3.73-3.56 (m, 2H), 3.39-3.34 (m, 4H), 1.10 (t, 6H, J=6.8Hz); HRMS (ESI+) m/z [M + H]⁺ calculated for C₂₁H₂₈N₈O₄: 457.2306; found: 457.2305.

### Example 16: Synthesis of (2R,3R,4S,5R)-2-{6-amino-2-{2-[(E)-3-ethoxy-4-hydroxy benzylidene]hydrazino}-9H-purin-9-yl}-5-(hydroxymethyl)tetrahydrofuran-3,4-diol (Compound 16)

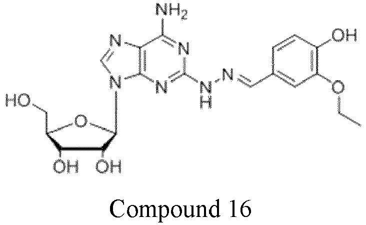

By referring to the method of step 1.4 in Example 1, and replacing 4-acetamidobenzaldehyde with ethyl vanillin, the title compound was obtained as 322 mg of white solid (Compound 16). m.p. 198°C; ¹H NMR (DMSO-*d₆*): δ (ppm) 10.43 (s, 1H), 9.19 (s, 1H), 7.99 (s, 1H), 7.94 (s, 1H), 7.66 (s, 1H), 7.04 (br, 2H), 6.92 (d, 1H, J=8.0Hz), 6.77 (d, 1H, J=8.4Hz), 5.75 (d, 1H, J=7.2Hz), 5.48 (d, 1H, J=6.4Hz), 5.44-5.43 (m, 1H), 5.14 (d, 1H, J=4.0Hz), 4.86-4.84 (m, 1H), 4.17 (s, 1H), 4.09 (q, 2H, J=6.8Hz), 4.00 (s, 1H), 3.72-3.52 (m, 2H), 1.37 (t, 3H, J=6.8Hz); HRMS (ESI+) m/z [M + H]⁺ calculated for C₁₉H₂₃N₇O₆: 446.1783; found: 446.1782.

### Example 17: Synthesis of (2R,3R,4S,5R)-2-{6-amino-2-{2-[(E)-4-morpholinobenzylidene] hydrazino}-9H-purin-9-yl}-5-(hydroxymethyl)tetrahydrofuran-3,4-diol (Compound 17)

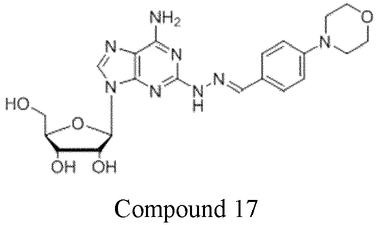

By referring to the method of step 1.4 in Example 1, and replacing 4-acetamidobenzaldehyde with 4-(morpholin-4-yl)benzaldehyde, the title compound was obtained as 348 mg of white solid (Compound 17). m.p. 186°C; ¹H NMR (DMSO-*d₆*): δ (ppm) 10.42 (s, 1H), 8.01 (s, 1H), 7.97 (s, 1H), 7.59 (d, 2H, J=8.4Hz), 7.02 (br, 2H), 6.95 (d, 2H, J=8.8Hz), 5.79 (d, 1H, J=6.4Hz), 5.46 (d, 1H, J=6.0Hz), 5.29-5.26 (m, 1H), 5.15 (d, 1H, J=4.0Hz), 4.69-4.65 (m, 1H), 4.21-4.18 (m, 1H), 3.97-3.95 (m, 1H), 3.74 (t, 4H, J=8.4Hz), 3.71-3.56 (m, 2H), 3.17 (t, 4H, J=8.8Hz); HRMS (ESI+) m/z [M + H]⁺ calculated for C₂₁H₂₆N₈O₅: 471.2099; found: 471.2100.

### Example 18: Synthesis of (2R,3R,4S,5R)-2-{6-amino-2-{2-[(E)-3,4,5-trimethoxy benzylidene]hydrazino}-9H-purin-9-yl}-5-(hydroxymethyl)tetrahydrofuran-3,4-diol (Compound 18)

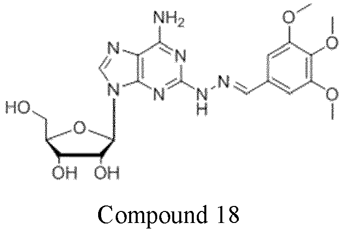

By referring to the method of step 1.4 in Example 1, and replacing 4-acetamidobenzaldehyde with 3,4,5-trimethoxybenzaldehyde, the title compound was obtained as 352 mg of white solid (Compound 18). m.p. 246°C; ¹H NMR (DMSO-*d₆*): δ (ppm) 10.68 (s, 1H), 8.01 (s, 1H), 7.98 (s, 1H), 7.16 (s, 2H), 7.09 (br, 2H), 5.75 (d, 1H, J=7.2Hz), 5.49-5.45 (m, 2H), 5.12 (s, 1H), 4.92-4.88 (m, 1H), 4.15 (s, 1H), 3.99 (s, 1H), 3.84 (s, 6H), 3.68 (s, 3H), 3.72-3.52 (m, 2H); HRMS (ESI+) m/z [M + H]⁺ calculated for C₂₀H₂₅N₇O₇: 476.1888; found: 476.1887.

### Example 19: Synthesis of (2R,3R,4S,5R)-2-{6-amino-2-{2-[(E)-4-(benzyloxy)-3-methoxy benzylidene]hydrazino}-9H-purin-9-yl}-5-(hydroxymethyl)tetrahydrofuran-3,4-diol (Compound 19)

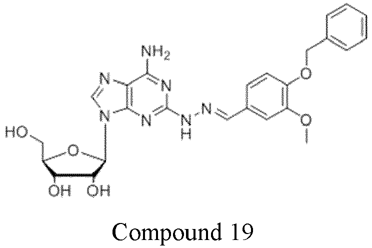

By referring to the method of step 1.4 in Example 1, and replacing 4-acetamidobenzaldehyde with 4-(benzyloxy)-3-methoxybenzaldehyde, the title compound was obtained as 281 mg of white solid (Compound 19). m.p. 146°C; ¹H NMR (DMSO-*d₆*): δ (ppm) 10.54 (s, 1H), 8.00 (s, 1H), 7.99 (s, 1H), 7.77 (s, 1H), 7.47-4.33 (m, 5H), 7.06 (br, 2H), 7.03 (s, 2H), 5.75 (d, 1H, J=7.2H), 5.48 (d, 1H, J=6.4Hz), 5.46 (d, 1H, J=4.0Hz), 5.13 (d, 1H, J=4.0Hz), 5.11 (s, 2H), 4.90-4.85 (m, 1H), 4.17-4.15 (m, 1H), 3.99 (s, 1H), 3.85 (s, 3H), 3.74-3.53 (m, 2H); HRMS (ESI+) m/z [M + H]⁺ calculated for C₂₅H₂₇N₇O₆: 522.2096; found: 522.2096.

### Example 20: Synthesis of 4-{E-{2-{6-amino-9-[(2R,3R,4S,5R)-3,4-dihydroxy-5-(hydroxymethyl)tetrahydrofuran-2-yl]-9H-purinylpyridin-2-yl}hydrazono}methyl}benzonitrile (Compound 20)

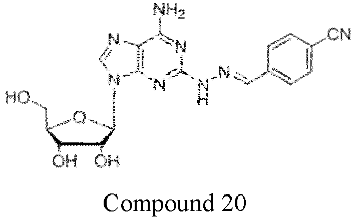

By referring to the method of step 1.4 in Example 1, and replacing 4-acetamidobenzaldehyde with 4-cyanobenzaldehyde, the title compound was obtained as 373 mg of white solid (Compound 20). m.p. 276°C; ¹H NMR (DMSO-*d₆*): δ (ppm) 10.99 (s, 1H), 8.11 (s, 1H), 8.07 (s, 1H), 7.91 (d, 2H, J=8.4Hz), 7.83 (d, 2H, J=8.0Hz), 7.13 (br, 2H), 5.81 (d, 1H, J=6.4Hz), 5.44 (d, 1H, J=6.4Hz), 5.28-5.26 (m, 1H), 5.15 (d, 1H, J= 4.8Hz), 4.72-4.67 (m, 1H), 4.23-4.20 (m, 1H), 3.98 (d, 1H, J=2.4Hz), 3.75-3.57 (m, 2H); HRMS (ESI+) m/z [M + H]⁺ calculated for C₁₈H₁₈N₈O₄: 411.1524; found: 411.1525.

### Example 21: Radioligand binding test

### 1) Experimental materials

[3H]CGS21680 (2-[p-(2-carboxyethyl)phenylethylamino]-5'-N-ethylcarboxamidoadenosine, [carboxy-1-ethyl-3H(N)]-;250µCi) was purchased from PerkinElmer Research Products (Boston, MA).

Cell membranes stably transfected with (human) A_{2A} adenosine receptors were prepared in HEK-293 cells. The cell membranes were obtained from PerkinElmer Research Products (Boston, MA).

CGS21680 (2-[p-(2-carboxyethyl)phenylethylamino]-5'-N-ethylcarboxamidoadenosine) was purchased from Selleck (Shanghai, CN).

All other reagents were of analytical grade and obtained from commercial sources.

### 2) Experimental method

The A_{2A} adenosine receptors used were all expressed in the cell membranes. The compound was diluted 3 times serially with DMSO (Solarbio, D8371-250ml) to generate compound source plates with 10 different concentrations (10µM, 3.3µM, 1.1µM, 0.37µM, 0.12µM, 0.0412µM, 0.0137µM, 0.0046µM, 0.0015µM, 0.0005µM), 250 nL of the compound was added to 384-well Opti-plate, sealed with parafilm. 20U hA_{2A} HEK-293 cell membrane was diluted with 1 mL assay buffer (50 mM Tris-HCl pH 7.4, 10 mM MgCl₂, 1 mM EDTA, 1µg/mL adenosine deaminase), 0.75 µCi [3H]CGS 21680 (final 25 nM) was added to the diluted cell membrane, and mixed well. 50 µL of the prepared dilution solution of cell membrane was transferred to the 384-well Opti-plate containing compound and incubated at 25°C for 90 minutes. 100 µL of 0.5% Polyethyleneimine solution (PEI) was added to UNIFILTER-96 GF/B filter plate, and soaked for 90 minutes at 4°C, then 500 µL of washing buffer/well (50mM Tris-HCl pH 7.4, 154mM NaCl) was transferred with Cell Harvester to wash the UNIFILTER-96 GF/B filter plate twice. The mixed system in the Opti-plate was transferred to the washed UNIFILTER-96 GF/B filter plate, 500 µL of washing buffer/well (50mM Tris-HCl pH 7.4, 154mM NaCl) was used to wash the UNIFILTER-96 GF/B filter plate 9 times. Incubation was performed in 37°C incubator for 3 minutes. 40 µL of ULTIMA GOLD scintillation solution (Perkin Elmer, Cat #77-16061) was added to each well, CPM (count per minute) value was read by a MicroBeta liquid scintillation counter (PerkinElmer). The specific binding percentage of [3H]CGS21680 was calculated according to the CPM value, % specific binding of [3H]CGS21680 = (CPMₛₐₘₚₗₑ- CPM_{Low Control}) / (CPM_{High control}- CPM_{Low Control}) ^{∗} 100, in which High Control was 0.5% DMSO, Low Control was 100 µM CGS21680. The IC₅₀ value was calculated by curve fitting according to the compound concentration and the specific binding percentage of [3H]CGS21680.

### 3) Experimental results

The inhibition constant (Kᵢ) value was calculated from the IC₅₀ value according to the Cheng and Prusoff equation, Kᵢ= IC₅₀/ (1 + [S] / Kₘ), wherein [S] was the concentration of radioligand (25nM) and Kₘ was dissociation constant (22 nM) of [3H]CGS21680 binding to human A_{2A}AR. Table 1 shows the inhibition constant Ki for Compounds 1 to 20 of the present application binding to A_{2A} adenosine receptor.

**Table 1: Test results of compounds binding to A_{2A} adenosine receptor**

| Compound | IC₅₀(nM) | K*ᵢ* (nM) |
|---|---|---|
| Compound 1 | 2840 | 1329 |
| Compound 2 | 3.9 | 1.8 |
| Compound 3 | >10,000 | 6276 |
| Compound 4 | 142.5 | 66.7 |
| Compound 5 | 11.7 | 5.5 |
| Compound 6 | 369.2 | 172.8 |
| Compound 7 | 1712 | 801.4 |
| Compound 8 | 444.9 | 208.3 |
| Compound 9 | 43.6 | 20.4 |
| Compound 10 | >10,000 | 6624.8 |
| Compound 11 | 130.9 | 61.3 |
| Compound 12 | 461.8 | 216.2 |
| Compound 13 | 479.3 | 224.4 |
| Compound 14 | 2790 | 1306 |
| Compound 15 | 13.7 | 6.4 |
| Compound 16 | 3465 | 1622 |
| Compound 17 | 1708 | 799.5 |
| Compound 18 | >10,000 | 5495 |
| Compound 19 | 216.4 | 101.3 |
| Compound 20 | 18.07 | 8.5 |

### Example 22: Adenosine receptor A_{2A} cAMP test

### 1) Experimental materials

Experimental reagents and consumables: DMEM/F12, G418, Penicillin-Streptomycin, Versene Solution, HEPES, Hank's Buffered Saline Solution, PBS (pH 7.4, 1×, sterile), FBS, BSA Stabilizer 7.5%, Rolipram, NECA were separately purchased from Gibico, Hyclone and Sigma. LANCE^{®} Ultra cAMP kit (Eu-cAMP tracer, Ulight-anti-cAMP reagent, cAMP detection buffer) and hADORA_{2A}-HEK293 cells were purchased from PerkinElmer Research Products (Boston, MA). All other reagents were of analytical grade and obtained from commercial sources. 384-well polypropylene microplate and 384-well solid white plate were purchased from Labcyte and Corning, respectively.

Experimental instruments: TECAN automated liquid handling workstation, Echo Acoustic Liquid Handler and EnVison multimode plate reader were purchased from TECAN, Labcyte and Envision, respectively.

### 2) Experimental method

The cells stably expressing human adenosine receptor A_{2A} (hADORA_{2A}-HEK293 cells) were cultured in DMEM/F12 medium containing 10% FBS, 1× Penicillin-Streptomycin and 400µg/ml G418 in a 37°C and 5% CO₂ environment. Before the experiment, the cells were digested with Versene solution, and the cells were collected by centrifugation at 200g and room temperature for 5 minutes, and finally resuspended with assay buffer (Hank's buffered saline solution, containing 5mM HEPES, 0.1% BSA stabilizer and 10µM Rolipram, pH 7.4). The TECAN automated liquid handling workstation was used to dilute the compound in a 384-well polypropylene microplate with DMSO to 11 concentration points in a 3-fold gradient to prepare the compound source plate, in which the 11 concentration points of the compound were 10mM, 3.33mM, 1.11mM., 0.37mM, 0.12mM, 0.041mM, 0.013mM, 4.57×10⁻³mM, 1.52×10⁻³mM, 5×10⁻⁴mM and 1.7×10⁻⁴mM. The Echo Acoustic Liquid Handler (Labcyte) was used to transfer the test compound from the compound source plate to an assay plate, in which the transfer volume of the compound was 10 nl/well. The hADORA_{2A}-HEK293 cell suspension was diluted with assay buffer to 30,000 cells/ml, and the cell suspension was transferred to the assay plate at a volume of 10 µl/well (300 cells/well). The assay plate was centrifuged at 150g for 1 minute and pre-incubated at room temperature for 30 minutes. The Eu-cAMP tracer working solution (Eu-cAMP tracer 40µl, cAMP detection buffer 1.96ml) (5µl/well) was added to the assay plate, and then the Ulight-anti-cAMP working solution (13µl of Ulight-anti-cAMP reagent and 1.95ml of cAMP detection buffer) (5µl/well) was added to the assay plate. The assay plate was rotated at 150g for 30 seconds and incubated at room temperature for 30 minutes. The EnVision multimode plate reader (PerkinElmer) was used determine the level of cyclic adenosine monophosphate in the final solution (λₑₓ= 320nm, λₑₘ= 665nm & 615nm). The EC₅₀ (nM) value of the compound interacting with the A_{2A} adenosine receptor to stimulate the production of cyclic adenosine monophosphate was calculated and the A_{2A} receptor agonist titer of the compound was expressed as the EC₅₀ (nM) value.

### 3) Experimental results

When the test compounds interacted with A_{2A}AR, the EC₅₀ (nM) values for stimulating the production of cyclic AMP were shown in Table 2. The results showed that Compounds 2, 5, 9, 15 and 20 prepared in the present application were all shown as hA_{2A}AR agonists. When Compounds 2, 5, 9, 15 and 20 interacted with A_{2A}AR, their inhibitory constant Ki and EC₅₀ values for stimulating cAMP production were basically in the same nanomolar range.

**Table 2: EC₅₀ value test results of compound A_{2A} agonist function determination**

| Compound | cAMP EC₅₀ (nM) |
|---|---|
| Compound 2 | 0.64 |
| Compound 5 | 4.1 |
| Compound 9 | 5.7 |
| Compound 15 | 17.9 |
| Compound 20 | 15.3 |

### Example 23: Animal experiment of blood-brain barrier disruption method

### 1) Experimental materials

Fluorescein-labeled dextran FITC-Dextran (CAS: 60842-46-8) with molecular weight of 10,000 MW was purchased from TCI (Shanghai) Development Co., Ltd.; PBS solution and experimental animal SD rats were obtained from commercial sources.

### 2) Experimental method

FITC-Dextran solutions with six concentration gradients (0.001, 0.01, 0.1, 1, 0.5, 10 µg/ml) were prepared with PBS, and standard curve of FITC-Dextran concentration was made by using microplate reader (λₑₓ= 490nm, λₑₘ= 520 nm). Additionally, 10 mg/ml FITC-Dextran solution was prepared, Compound 5 was added to PBS solution to prepare 1mg/ml solution. 1ml of 10mg/ml FITC-Dextran solution and 1ml of 1mg/ml Compound 5 in PBS solution were taken and mixed to prepare administration solvent. 1ml of 10mg/ml FITC-Dextran solution and 1ml of PBS solution were taken and mixed to prepare blank control solution. 6 SD rats were injected with 2ml of the administration solvent via tail vein, respectively, while another 6 SD rats were injected with 2ml of the blank control solution via tail vein. After 30 minutes, brain tissues were taken out from all SD rats, homogenized and centrifuged at 10,000 rpm for 15 minutes, and the supernatants were taken for later test. The solution to be tested was subjected to fluorescence detection by using the microplate reader (λₑₓ = 490 nm, λₑₘ = 520 nm).

### 3) Experimental results

The measured fluorescence value by the microplate reader was converted into the corresponding FITC-Dextran average concentration according to the obtained standard curve of FITC-Dextran concentration. The results were shown in Table 3. The results showed that the macromolecule FITC-Dextran itself could not pass through the blood-brain barrier, while FITC-Dextran to which Compound 5 was added could enter the brain through the BBB, indicating that Compound 5 could open the blood-brain barrier.

**Table 3: Results of FITC-Dextran concentration detection in brain of SD rats**

| | PBS solution containing Compound 5 | Blank control solution |
|---|---|---|
| FITC-Dextran concentration (µg/ml) | 0.11 | 0.020 |

Although the specific embodiments of the present application have been described in details, those skilled in the art will understand that according to all the teachings that have been disclosed, various modifications and substitutions can be made to those details, and these changes are all within the scope of protection of the present application. The full scope of the present application is given by the appended claims and any equivalents thereof. The publications and patent documents cited in the present application are incorporated herein by reference.

## Claims

1. A compound represented by the general Formula (I), a stereoisomer, a pharmaceutically acceptable salt, a pharmaceutically acceptable hydrate or solvate, or a pharmaceutically acceptable ester thereof, 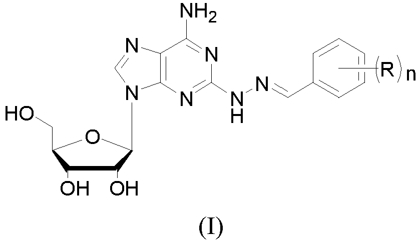 wherein,
n is 1, 2, 3, 4 or 5;
R represents a substituent attached to the benzene ring, and each R is independently selected from the group consisting of hydrogen, halogen, cyano, benzyloxy, halogenated benzyloxy, C₁₋₆ alkyl, halogenated C₁₋₆ alkyl, C₁₋₆ alkoxy, hydroxyl, C₁₋₆ alkylamino, di(C₁₋₆ alkyl)amino, anilino, diphenylamino, phenylamino, -NHC(O)R¹⁰, aryl, heteroaryl, cycloalkyl, and heterocycloalkyl, wherein R¹⁰ is C₁₋₆ alkyl.

2. The compound, a stereoisomer, a pharmaceutically acceptable salt, a pharmaceutically acceptable hydrate or solvate, or a pharmaceutically acceptable ester thereof according to claim 1, wherein n is 1, 2, or 3.

3. The compound, a stereoisomer, a pharmaceutically acceptable salt, a pharmaceutically acceptable hydrate or solvate, or a pharmaceutically acceptable ester thereof according to claim 1, wherein each R is independently selected from the group consisting of hydrogen, fluorine, chlorine, bromine, iodine, cyano, benzyloxy, fluorobenzyloxy, C₁₋₄ alkyl, halogenated C₁₋₄ alkyl, C₁₋₄ alkoxy, hydroxyl, C₁₋₄ alkylamino, di(C₁₋₄ alkyl)amino, phenylamino, diphenylamino, - NHC(O)R¹⁰, phenyl, pyridyl, pyrrolidinyl, cyclopentyl, cyclohexyl, morpholinyl, and imidazolyl, wherein R¹⁰ is C₁₋₄ alkyl.

4. The compound, a stereoisomer, a pharmaceutically acceptable salt, a pharmaceutically acceptable hydrate or solvate, or a pharmaceutically acceptable ester thereof according to claim 1, wherein each R is independently selected from the group consisting of hydrogen, fluorine, chlorine, bromine, iodine, cyano, benzyloxy, fluorobenzyloxy, methyl, ethyl, n-propyl, isopropyl, n-butyl, isobutyl, tert-butyl, n-pentyl, tert-pentyl, neopentyl, hexyl, trifluoromethyl, difluoromethyl, fluoromethyl, methoxy, ethoxy, n-propoxy, isopropoxy, n-butoxy, tert-butoxy, sec-butoxy, n-pentyloxy, n-hexyloxy, hydroxyl, methylamino, ethylamino, propylamino, butylamino, dimethylamino, diethylamino, dipropylamino, dibutylamino, phenylamino, diphenylamino, acetamido, formylamino, propionamido, phenyl, pyridyl, pyrrolidinyl, cyclopentyl, cyclohexyl, morpholinyl, and imidazolyl.

5. The compound, a stereoisomer, a pharmaceutically acceptable salt, or a pharmaceutically acceptable hydrate thereof according to claim 1, wherein each R is independently selected from the group consisting of hydrogen, methoxy, ethoxy, acetyl, acetamido, benzyloxy, trifluoromethyl, diphenylamino, 4-fluorobenzyloxy, chlorine, pyridin-2-yl, phenyl, pyrrolidin-1-yl, 1H-imidazol-1-yl, propoxy, diethylamino, hydroxyl, morpholin-4-yl, and cyano.

6. The compound, a stereoisomer, a pharmaceutically acceptable salt, a pharmaceutically acceptable hydrate or solvate, or a pharmaceutically acceptable ester thereof according to claim 1, wherein the compound has a structure represented by Formula 1-1, 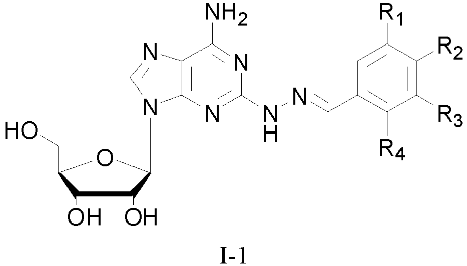
wherein, R₁, R₂, R₃, R₄ are each independently selected from the group consisting of hydrogen, halogen, cyano, benzyloxy, halogenated benzyloxy, C₁₋₆ alkyl, halogenated C₁₋₆ alkyl, C₁₋₆ alkoxy, hydroxyl, C₁₋₆ alkylamino, di(C₁₋₆ alkyl)amino, anilino, diphenylamino, phenylamino, -NHC(O)R¹⁰, aryl, heteroaryl, cycloalkyl, and heterocycloalkyl, wherein R¹⁰ is C₁-₆ alkyl;
for example, R₁, R₂, R₃, R₄ are each independently selected from the group consisting of hydrogen, fluorine, chlorine, bromine, iodine, cyano, benzyloxy, fluorobenzyloxy, C₁₋₄ alkyl, halogenated C₁₋₄ alkyl, C₁₋₄ alkoxy, hydroxyl, C₁₋₄ alkylamino, di(C₁₋₄ alkyl)amino, phenylamino, diphenylamino, -NHC(O)R¹⁰, phenyl, pyridyl, pyrrolidinyl, cyclopentyl, cyclohexyl, morpholinyl, and imidazolyl, wherein R¹⁰ is C₁₋₄ alkyl;
for example, R₁, R₂, R₃, R₄ are each independently selected from the group consisting of hydrogen, fluorine, chlorine, bromine, iodine, cyano, benzyloxy, fluorobenzyloxy, methyl, ethyl, n-propyl, isopropyl, n-butyl, isobutyl, tert-butyl, n-pentyl, tert-pentyl, neopentyl, hexyl, trifluoromethyl, difluoromethyl, fluoromethyl, methoxy, ethoxy, n-propoxy, isopropoxy, n-butoxy, tert-butoxy, sec-butoxy, n-pentyloxy, n-hexyloxy, hydroxyl, methylamino, ethylamino, propylamino, butylamino, dimethylamino, diethylamino, dipropylamino, dibutylamino, phenylamino, diphenylamino, acetamido, formylamino, propionamido, phenyl, pyridyl, pyrrolidinyl, cyclopentyl, cyclohexyl, morpholinyl, and imidazolyl;
for example, R₁, R₂, R₃, R₄ are each independently selected from the group consisting of hydrogen, methoxy, ethoxy, acetamido, benzyloxy, trifluoromethyl, diphenylamino, 4-fluorobenzyloxy, chlorine, pyridin-2-yl, phenyl, pyrrolidin-1-yl, 1H-imidazol-1-yl, propoxy, diethylamino, hydroxyl, morpholin-4-yl, and cyano;
for example, R₁ is hydrogen or methoxy;
for example, R₂ is hydrogen, methoxy, acetamido, benzyloxy, trifluoromethyl, diphenylamino, 4-fluorobenzyloxy, chlorine, pyridin-2-yl, phenyl, pyrrolidin-1-yl, 1H-imidazol-1-yl, propoxy, diethylamino, hydroxyl, morpholin-4-yl, or cyano;
for example, R₃ is hydrogen, benzyloxy, trifluoromethyl, ethoxy or methoxy;
for example, R₄ is hydrogen, trifluoromethyl or cyano;
for example, R₁ and R₄ are each independently hydrogen; R₂ is di(C₁₋₆ alkyl)amino, C₁₋₆ alkylamino, benzyloxy, halogenated benzyloxy, phenyl, halogenated phenyl or cyano; R₃ is hydrogen or benzyloxy;
for example, R₁, R₃, and R₄ are each independently hydrogen; R₂ is di(C₁₋₆ alkyl)amino, C₁₋₆ alkylamino, benzyloxy, halogenated benzyloxy, phenyl, halogenated phenyl or cyano.

7. The compound, a stereoisomer, a pharmaceutically acceptable salt, a pharmaceutically acceptable hydrate or solvate, or a pharmaceutically acceptable ester thereof according to claim 1, wherein the compound is selected from the group consisting of:
N-{4-{(E)-{2-{6-amino-9-[(2R,3R,4S,5R)-3,4-dihydroxy-5-(hydroxymethyl)tetrahydrofuran-2-yl]-9H-purin-2-yl}hydrazono}methyl}phenyl}acetamide;
(2R,3R,4S,5R)-2-{6-amino-2-{2-[(E)-3,4-bis(benzyloxy)benzylidene]hydrazino}-9H-pruin-9-yl}-5-(hydroxymethyl)tetrahydrofuran-3,4-diol;
(2R,3R,4S,5R)-2-{6-amino-2-{2-[(E)-2,4-bis(trifluoromethyl)benzylidene]hydrazino}-9H-pruin-9-yl}-5-(hydroxymethyl)tetrahydrofuran-3,4-diol;
(2R,3R,4S,5R)-2-{6-amino-2-{2-[(E)-4-(diphenylamino)benzylidene]hydrazino}-9H-pruin-9-yl}-5-(hydroxymethyl)tetrahydrofuran-3,4-diol;
(2R,3R,4S,5R)-2-{6-amino-2-{2-{(E)-4-[(4-fluorobenzyl)oxy]benzylidene}hydrazino}-9H-purin-9-yl}-5-(hydroxymethyl)tetrahydrofuran-3,4-diol;
(2R,3R,4S,5R)-2-{6-amino-2-{2-[(E)-3-(benzyloxy)benzylidene]hydrazino}-9H-purin-9-yl}-5-(hydroxymethyl)tetrahydrofuran-3,4-diol;
(2R,3R,4S,5R)-2-{6-amino-2-{2-[(E)-4-chloro-3-(trifluoromethyl)benzylidene]hydrazino}-9H-purin-9-yl}-5-(hydroxymethyl)tetrahydrofuran-3,4-diol;
(2R,3R,4S,5R)-2-{6-amino-2-{2-[(E)-4-(pyridin-2-yl)benzylidene]hydrazino)-9H-purin-9-yl}-5-(hydroxymethyl)tetrahydrofuran-3,4-diol;
(2R,3R,4S,5R)-2-{2-{2-[(E)-[1,1'-biphenyl]-4-yl-methylene]hydrazino}-6-amino-9H-purin-9-yl}-5-(hydroxymethyl)tetrahydrofuran-3,4-diol;
(2R,3R,4S,5R)-2-{6-amino-2-{2-[(E)-4-(pyrrolidin-1-yl)benzylidene]hydrazino)-9H-pruin-9-yl}-5-(hydroxymethyl)tetrahydrofuran-3,4-diol;
(2R,3R,4S,5R)-2-{6-amino-2-{2-[(E)-4-(trifluoromethyl)benzylidene]hydrazino}-9H-purin-9-yl}-5-(hydroxymethyl)tetrahydrofuran-3,4-diol;
(2R,3R,4S,5R)-2-{2-{2-[(E)-4-(1H-imidazol-1-yl)benzylidene]hydrazino}-6-amino-9H-pruin-9-yl}-5-(hydroxymethyl)tetrahydrofuran-3,4-diol;
(2R,3R,4S,5R)-2-{6-amino-2-{2-[(E)-4-propoxybenzylidene]hydrazino}-9H-purin-9-yl}-5-(hydroxymethyl)tetrahydrofuran-3,4-diol;
2-{(E)-{2-{6-amino-9-[(2R,3R,4S,5R)-3,4-dihydroxy-5-(hydroxymethyl)tetrahydrofuran-2-yl]-9H-purinylpyridin-2-yl}hydrazono}methyl}benzonitrile;
(2R,3R,4S,5R)-2-{6-amino-2-{2-[(E)-4-(diethylamino)benzylidene]hydrazino}-9H-purin-9-yl}-5-(hydroxymethyl)tetrahydrofuran-3,4-diol;
(2R,3R,4S,5R)-2-{6-amino-2-{2-[(E)-3-ethoxy-4-hydroxybenzylidene]hydrazino}-9H-purin-9-yl}-5-(hydroxymethyl)tetrahydrofuran-3,4-diol;
(2R,3R,4S,5R)-2-{6-amino-2-{2-[(E)-4-morpholinobenzylidene]hydrazino}-9H-purin-9-yl}-5-(Hydroxymethyl)tetrahydrofuran-3,4-diol;
(2R,3R,4S,5R)-2-{6-amino-2-{2-[(E)-3,4,5-trimethoxybenzylidene]hydrazino}-9H-purin-9-yl}-5-(hydroxymethyl)tetrahydrofuran-3,4-diol;
(2R,3R,4S,5R)-2-{6-amino-2-{2-[(E)-4-(benzyloxy)-3-methoxybenzylidene]hydrazino}-9H-purin-9-yl}-5-(hydroxymethyl)tetrahydrofuran-3,4-diol; and
4-{E-{2-{6-amino-9-[(2R,3R,4S,5R)-3,4-dihydroxy-5-(hydroxymethyl)tetrahydrofuran-2-yl]-9H-purinylpyridin-2-yl}hydrazono}methyl}benzonitrile.

8. A method for preparing the compound represented by the general Formula (I), a stereoisomer, a pharmaceutically acceptable salt, a pharmaceutically acceptable hydrate or solvate, or a pharmaceutically acceptable ester thereof according to any one of claims 1 to 5, comprising: 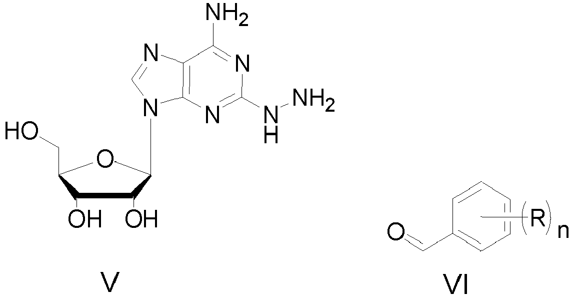
reacting a compound represented by Formula V with a substituted benzaldehyde represented by Formula VI to obtain the compound represented by the general Formula (I), wherein the definitions of R and n are the same as those described in claims 1 to 4,
preferably, the compound represented by Formula V reacts with the substituted benzaldehyde represented by Formula VI in a methanol solution under microwave heating at 70°C to 90°C;
preferably, the compound represented by Formula V is produced by the hydrazinolysis of a compound represented by Formula IV with hydrazine hydrate at 40°C to 60°C; 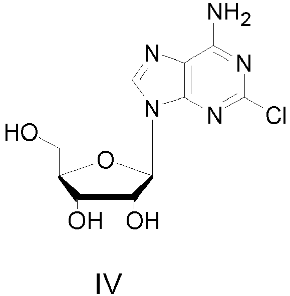
preferably, the compound of Formula IV is produced by the ammonolysis of a compound represented by Formula III in a solution of ammonia in methanol at 90°C to 110°C; 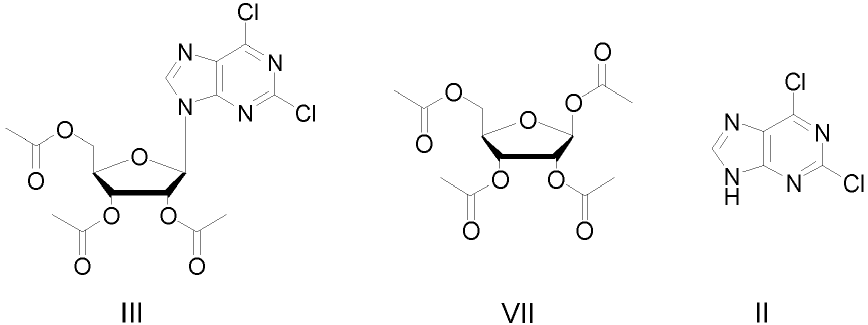
preferably, the compound represented by Formula III is produced by a substitution reaction of a compound represented by Formula VII with a compound represented by Formula II in the presence of tin tetrachloride as a catalyst at 110°C to 130°C.

9. A pharmaceutical composition, comprising at least one of the compound, a stereoisomer, a pharmaceutically acceptable salt, a pharmaceutically acceptable hydrate or solvate, or a pharmaceutically acceptable ester thereof according to any one of claims 1 to 7, and one or more pharmaceutically acceptable carriers or excipients.

10. Use of the compound, a stereoisomer, a pharmaceutically acceptable salt, a pharmaceutically acceptable hydrate or solvate, or a pharmaceutically acceptable ester thereof according to any one of claims 1 to 7, or the pharmaceutical composition according to claim 9 in the manufacture of a medicament as an A_{2A} adenosine receptor agonist, or
in the manufacture of a medicament for the prevention and/or treatment of a human pathological condition or symptom, wherein the human pathological condition or symptom is related to the activity of A_{2A} adenosine receptor, and the prevention or treatment of the human pathological condition or symptom requires the activation of A_{2A} adenosine receptor.

11. The use according to claim 10, wherein the human pathological condition or symptom is selected from the group consisting of: autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, foundrymen's disease, glaucoma, ocular hypertension, spinal cord injury, myocardial infarction, and acute myocardial infarction.

12. Use of the compound, a stereoisomer, a pharmaceutically acceptable salt, a pharmaceutically acceptable hydrate or solvate, or a pharmaceutically acceptable ester thereof according to any one of claims 1 to 7, or the pharmaceutical composition according to claim 9 in the manufacture of a medicament for diagnosing a human abnormal myocardial perfusion, or in the manufacture of a medicament as coronary vasodilator.

13. Use of the compound, a stereoisomer, a pharmaceutically acceptable salt, a pharmaceutically acceptable hydrate or solvate, or a pharmaceutically acceptable ester thereof according to any one of claims 1 to 7, or the pharmaceutical composition according to claim 9 in the manufacture of a medicament for increasing the permeability of blood-brain barrier in a subject receiving a therapeutic drug, wherein the subject benefits from the increased permeability of blood-brain barrier for delivering the therapeutic drug across the blood-brain barrier.

14. The use according to claim 13, wherein the therapeutic drug is selected from the group consisting of: drug for treating disease or disorder of central nervous system, antidote to nerve agent, and drug for treating glioma.

15. A pharmaceutical composition, comprising:
at least one of the compound, a stereoisomer, a pharmaceutically acceptable salt, a pharmaceutically acceptable hydrate or solvate, or a pharmaceutically acceptable ester thereof according to any one of claims 1 to 7, and
a drug that needs to cross blood-brain barrier, which is selected from the group consisting of drug for treating disease or disorder of central nervous system, antidote to nerve agent, and drug for treating glioma, and
one or more pharmaceutically acceptable carriers or excipients.

16. A method for the prevention and/or treatment of a human pathological condition or symptom, comprising administering to a patient in need thereof a therapeutically effective amount of at least one of the compound, a stereoisomer, a pharmaceutically acceptable salt, a pharmaceutically acceptable hydrate or solvate, or a pharmaceutically acceptable ester thereof according to any one of claims 1 to 7, or the pharmaceutical composition according to claim 9, wherein the human pathological condition or symptom is related to the activity of A_{2A} adenosine receptor, and the prevention or treatment of the human pathological condition or symptom requires agonizing the activity of A_{2A} adenosine receptor;
preferably, the human pathological condition or symptom is selected from the group consisting of: autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, foundrymen's disease, glaucoma, ocular hypertension, spinal cord injury, myocardial infarction, and acute myocardial infarction.

17. The compound represented by the general Formula (I), a stereoisomer, a pharmaceutically acceptable salt, a pharmaceutically acceptable hydrate or solvate, or a pharmaceutically acceptable ester according to any one of claims 1 to 7, for use in the prevention and/or treatment of a human pathological condition or symptom, wherein the human pathological condition or symptom is related to the activity of A_{2A} adenosine receptor, and the prevention or treatment of the human pathological condition or symptom requires agonizing the activity of A_{2A} adenosine receptor,
preferably, the human pathological condition or symptom is selected from the group consisting of: autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, foundrymen's disease, glaucoma, ocular hypertension, spinal cord injury, myocardial infarction, and acute myocardial infarction.

18. The compound represented by the general Formula (I), a stereoisomer, a pharmaceutically acceptable salt, a pharmaceutically acceptable hydrate or solvate, or a pharmaceutically acceptable ester thereof according to any one of claims 1 to 7, for use as an A_{2A} adenosine receptor agonist or coronary vasodilator, or
for use in the diagnosis of a human abnormal myocardial perfusion, or
for use in increasing the permeability of blood-brain barrier in a subject receiving a therapeutic drug, the subject benefits from the increased permeability of blood-brain barrier for delivering the therapeutic drug across the blood-brain barrier,
preferably, the therapeutic drug is selected from the group consisting of: drug for treating disease or disorder of central nervous system, antidote to nerve agent, and drug for treating glioma.

19. A method for the diagnosis of a human abnormal myocardial perfusion, comprising administering to a subject in need thereof a diagnostically effective amount of the compound, a stereoisomer, a pharmaceutically acceptable salt, a pharmaceutically acceptable hydrate or solvate, or a pharmaceutically acceptable ester thereof according to any one of claims 1 to 7, or the pharmaceutical composition according to claim 9.

20. A method for increasing the permeability of blood-brain barrier in a subject receiving a therapeutic drug, the method comprising administering to the subject an effective amount of the compound, a stereoisomer, a pharmaceutically acceptable salt, a pharmaceutically acceptable hydrate or solvate, or a pharmaceutically acceptable ester thereof according to any one of claims 1 to 7, or the pharmaceutical composition according to claim 9, wherein the subject benefits from the increased permeability of blood-brain barrier for delivering the therapeutic drug across the blood-brain barrier.

21. Preferably, the therapeutic drug is selected from the group consisting of: drug for treating disease or disorder of central nervous system, antidote to nerve agent, and drug for treating glioma.
